# EUROPEAN PATENT SPECIFICATION

(11) **EP 2 318 034 B1**
(45) Date of publication and mention of the grant of the patent: **22.03.2017**
(21) Application number: 09798310.0
(22) Date of filing: 15.07.2009
(51) Int. Cl.: A61K 38/18, A61K 38/20, A61K 8/64, A61P 17/02, A61Q 19/00

(54) **CP CONDITIONED MEDIUM FOR USE IN WOUND HEALING**
CP KONDIZIONIERTES MEDIUM ZUR VERWENDUNG IN DER WUNDHEILUNG
PC MILIEU CONDITIONNÉ POUR L'UTILISATION DANS LA CICATRISATION

(30) Priority: 15.07.2008 US 135026 P
(43) Date of publication of application: 11.05.2011
(73) Proprietor: Thanos, Christopher, Cumberland, RI 02864 (US); Bintz, Briannan, Cumberland, RI 02864 (US)
(72) Inventor: Thanos, Christopher, Cumberland, RI 02864 (US); Bintz, Briannan, Cumberland, RI 02864 (US)
(74) Representative: Helbig, Christian
(86) International application number: PCT/US2009/004129
(87) International publication number: WO 2010/008573

(56) References cited:
- WO-A1-2006/112734
- WO-A1-2006/132548
- WO-A2-00/66188
- US-A1- 2004 213 768
- US-A1- 2008 119 909
- KUROYANAGI YOSHIMITSU ET AL: "Establishment of banking system for allogeneic cultured dermal substitute", ARTIFICIAL ORGANS, BLACKWELL SCIENTIFIC PUBLICATIONS, INC., BOSTON, US, vol. 28, no. 1, 1 January 2004 (2004-01-01), pages 13-21, XP009160358, ISSN: 0160-564X, DOI: 10.1111/J.1525-1594.2004.07318.X [retrieved on 2004-01-04]
- MEHTA SAMIR ET AL: "Platelet rich concentrate: basic science and current clinical applications", JOURNAL OF ORTHOPAEDIC TRAUMA, NEW YORK, NY, US, vol. 22, no. 6, 1 July 2008 (2008-07-01), pages 433-438, XP009160357, ISSN: 0890-5339
- CHOUKROUN ET AL: "Platelet-rich fibrin (PRF): A second-generation platelet concentrate. Part IV: Clinical effects on tissue healing", ORAL SURGERY, ORAL MEDICINE, ORAL PATHOLOGY, ORAL RADIOLOGY AND ENDODONTICS, MOSBY-YEAR BOOK, ST. LOUIS, MO, US, vol. 101, no. 3, 1 March 2006 (2006-03-01) , pages E56-E60, XP005339146, ISSN: 1079-2104, DOI: 10.1016/J.TRIPLEO.2005.07.011

## Description

### FIELD OF THE INVENTION

The invention relates to compositions for improving wound healing in particular for preventing scar formation and thus loss of function that can occur in injured tissues during the natural wound healing process. Particularly, although by no means exclusively, the invention relates to the healing of chronic wounds such as diabetic ulcers.

### BACKGROUND

A wound is a disruption of tissue integrity that is typically associated with a degenerative or traumatic loss of biological substance. Simple wounds include cuts and scrapes to the skin whilst deeper injuries to the muscle tissue, skeletal system or the inner organs are defined as complicated wounds¹.

Every wound undergoes a similar reparative process independent of the wound type or the degree of tissue damage^{1, 2, 3}. Three distinct phases of wound healing are recognised. Firstly the inflammatory or exudative phase for the detachment of deteriorated tissue and for wound cleansing; secondly a proliferative phase for the development of granulation tissue; and thirdly a differentiation or regeneration phase for maturation and scar formation¹.

The inflammatory phase is characterised by hemostasis and inflammation. After injury to tissue occurs, the cell membranes, damaged from the wound formation, release thromboxane A₂ and prostoglandin 2-alpha, potent vasoconstrictors. This initial response helps to limit haemorrhage. Capillary vasodilation then occurs and inflammatory cells (platelets, neutrophils, leukocytes, macrophages, and T lymphocytes), migrate to the wound site. In particular, neutrophil granulocytes play a central role in wound cleansing via phagocytosis. The next cells present in the wound are the leukocytes and macrophages. The macrophages in particular, are essential for wound healing. Numerous enzymes and cytokines are secreted by the macrophage, including collagenases, which debride the wound; interleukins and tumor necrosis factor (TNF), which stimulate fibroblasts (to produce collagen) and promote angiogenesis; and transforming growth factor (TGF), which stimulates keratinocytes². This step marks the transition into the process of tissue reconstruction, i.e. the proliferation phase.

The proliferation phase is characterised by epithelialisation, angiogenesis, granulation tissue formation, and collagen deposition. Angiogenesis stimulated by multiple factors, such as TNF alpha, is essential to deliver nutrients into and around the wound site and is critical for efficient wound healing. Granulation tissue formation is a complex event involving leukocytes, histiocytes, plasma cells, mast cells, and in particular fibroblasts, that promote tissue growth through the production of collagen. The exact steps and mechanism of control of the proliferation phase are unknown. Some cytokines involved include platelet derived growth factor (PDGF), insulin like growth factor (IGF) and epidermal growth factor (EGF). All are necessary for collagen formation².

The final phase of the classical wound healing cascade is the differentiation phase. The wound undergoes contraction and the granulation tissue becomes increasingly depleted of fluids and blood vessels, begins to strengthen, and undergoes remodelling to form scar tissue. Where the wound involves damage to the skin, the final stage in wound healing is epithelialisation, whereby epidermal cells migrate to resurface the denuded area. Where a wound includes damage to skeletal muscle, new muscle cells are laid down (in addition to granulation tissue in the proliferative phase) via satellite cells which differentiate to form myoblasts⁴. In the final stage of wound healing the myoblasts differentiate to form myotubes which mature and are incorporated into muscle fibres. Whilst this process results in the gain of some muscle function at the wound site, muscle wounds invariably result in loss of muscle tissue, scarring and loss of original muscle function.

In some large open wounds, such as in chronic wounds and ulcers, the normal reparative process is interrupted and the healing process is prolonged or incomplete.

Many factors can contribute towards poor wound healing, including local causes such as wound infection, tissue hypoxia, repeated trauma, the presence of debris and necrotic tissue and systemic causes such as diabetes mellitus, malnutrition, immunodeficiency, and the use of some medications.

For example, diabetic ulcers are the most common cause of foot and leg amputation. In patients with type I and II diabetes, the incidence rate of developing foot ulcers is approximately 2% per year and as such ulcers are prone to infection and notoriously difficult to heal.

Current treatments for acute and chronic tissue wounds include methods of improving circulation and thus oxygen and nutrient delivery to a wound site to improve healing times. This may be achieved mechanically, such as by using ultrasound treatment, magnetic and electrical simulation, whirlpool therapy and oxygen therapy. However, whilst these therapies are effective in stimulating and even accelerating the wound healing process, they still result in functional and/or cosmetic impairment at the wound site⁵.

New therapies are currently being investigated using cytokines and growth factors such as TGF-beta, EGF and IGF-1. TNF agonists and antagonists may also be useful in modifying angiogenesis, thus providing significant potential to improve the healing process directly. However, to date growth factors have had a limited role in clinical practice. The only currently available commercial product is PDGF (Regranex) which has been shown in some but not all studies to provide a modest improvement in healing time, but which has not been successful in improving the cosmetic or functional properties of healed tissue².

Thus, there is a need to provide new wound healing therapies which are able to control the wound healing process in both acute and chronic wounds so that new tissue would replace damaged tissue with no functional or cosmetic impairment.

It is an object of the present invention to go some way towards fulfilling this need and/or to provide the public with a useful choice.

### SUMMARY OF THE INVENTION

The present invention is defined by the claims.

Surprisingly, proteins secreted by choroid plexus (CP) cells have been shown for the first time to be useful in accelerating the wound healing process, particularly in reducing scarring in full thickness wounds and in the healing of chronic wounds.

Accordingly, the present disclosure provides a therapeutic composition comprising isolated or purified CP secreted proteins, wherein the CP secreted proteins are isolated or purified from CP conditioned media.

The present disclosure also provides a therapeutic composition comprising encapsulated CP cells secreting proteins useful for wound healing.

The present invention also provides a therapeutic composition comprising CP conditioned media produced by the method according to claim 1.

The present disclosure also provides a cosmetic composition comprising CP conditioned media.

The present disclosure also provides a cosmetic composition comprising encapsulated CP cells.

The therapeutic and cosmetic compositions of the present disclosure comprising the invention may be combined with a pharmaceutically acceptable carrier or excipient to provide pharmaceutical compositions.

The compositions of the invention may additionally comprise one or more compounds to give an additive or synergistic effect, wherein the compounds are selected from the group consisting of glucocorticosteroids, such as prednisone and methylprednisone, nonsteroidal anti-inflammatory drugs (NSAIDS), growth factors (PDGF, EGF, IGF, VEGF, TGF-Beta, and others), antibiotics, trophic or tropic compounds, peptides, mimetics, oligonucleotides, antibodies, inhibitors, as well as first and second generation anti-TNFα agents.

The compositions of the invention may be formulated for topical, cutaneous, subcutaneous, intradermal, oral, intranasal, intraperitoneal, intramuscular, intraosseous, intraocular, or intravenous administration. Preferably, the composition is formulated for topical administration.

The present invention also provides a method of improving tissue wound healing comprising the step of administering an effective amount of the compositions of the invention to a patient in need thereof. The present disclosure comprising the invention also provides a method of treating a wound in a patient comprising the step of administering an effective amount of the compositions of the invention to a patient in need thereof. The present disclosure comprising the invention also provides a method of promoting wound healing comprising the step of administering an effective amount of the compositions of the invention to a patient in need thereof. The present disclosure comprising the invention also provides a method of modulating the accumulation of fibroblasts at a wound site in a patient comprising the step of administering an effective amount of the compositions of the invention to a patient in need thereof. The present disclosure comprising the invention also provides a method of modulating the accumulation of collagen at a wound site in a patient comprising the step of administering an effective amount of the compositions of the invention to a patient in need thereof The invention may be useful in both animal and human wound healing.

Wound healing is improved in a human or animal patient via one or more of the following mechanisms:
(a) a decrease in the time of wound recovery;
(b) a restoration of advanced dermal or epidermal features
(c) an acceleration and increase in the inflammatory response; and
(d) a decrease or inhibition of scar tissue formation,
thereby resulting in improved functionality and cosmetic appearance of the treated tissue.

The proteins expressed by the CP of the invention comprise a mixture (e.g., two or more; any combination thereof) of VEGF, CTGF, PDGF, BMP-7, FGF-2, TGF-β, TGF-α, IL8/CXC chemokines, IGF-2, Cystatin, beta-2-microglobulin, Pleiotrophic factor β, PTHLH and other growth factors and extracellular matrix factors.

The present disclosure also provides for the use of CP secreted proteins isolated or purified from CP conditioned media, in the manufacture of a medicament for improving wound healing in a patient in need thereof.

The present disclosure also provides the use of CP secreted proteins isolated or purified from CP conditioned media, in the manufacture of a medicament for a cosmetic treatment of skin.

The present invention provides CP conditioned produced by the method of claim 1, for use in improving wound healing in a patient in need thereof.

The present disclosure also provides for the use of CP conditioned for a cosmetic treatment of skin.

The present disclosure also provides for the use of encapsulated CP cells, in the manufacture of a medicament for improving wound healing in a patient in need thereof.

The disclosure further provides for the use of encapsulated CP cells, in the manufacture of a medicament for a cosmetic treatment of skin.

The medicament may be formulated for local or systemic administration, for example, the medicament may be formulated for topical administration to an external wound site, or may be formulated for injection to an internal wound site. For cosmetic treatments, the medicament is preferably formulated for topical administration.

Another instance is a therapeutic composition comprising two or more isolated or purified choroid plexus (CP) secreted proteins (e.g., those delineated herein), and a clinically acceptable superficial wound treatment article (e.g., negative pressure therapy devices, bandages, films, adhesives, and the like).

The present disclosure further provides isolated or purified CP secreted proteins, CP conditioned media or encapsulated CP cells for use in a method of improving wound healing, or for cosmetic skin treatment, in a patient in need thereof.

The present disclosure also provides a method of manufacturing a composition comprising CP secreted proteins, comprising the steps:
a) culturing CP cells in vitro;
b) extracting CP conditioned media;
c) lyophilizing the CP conditioned media; and
d) optionally dialysing CP conditioned media, either before or after step c).

The lyophilized (and optionally dialysed) CP conditioned media may be combined with a suitable pharmaceutical carrier or excipient to produce a pharmaceutical composition.

The present disclosure also provides a composition manufactured by the method of the invention (e.g., those delineated herein).

The present disclosure comprising the invention provides a method of modulating the profile of the CP secreted proteins from by CP cells in vitro to increase the amount of proteins that are effective at increasing wound healing, said method comprising the steps:
a) culturing CP cells in vitro in suitable basal media;
b) adding media containing matrix proteins in an amount sufficient to initiate the formation of CP capsuloids,
wherein said capsuloids comprise a significantly higher levels of protein secretory vesicles when compared to CP cells grown in basal media alone (e.g., without further steps, without further additional agents, without step (b)).

The CP conditioned media extracted by this method contains a higher amount of VEGF and other CP secreted proteins useful in accelerating wound healing.

The present disclosure also provides compositions comprising CP proteins secreted by CP capsuloids containing modulated levels of secreted proteins, said capsuloids prepared by the methods of the invention.

The invention will now be described in more detail with reference to the figures of the accompanying drawings in which:

### BRIEF DESCRIPTION OF THE FIGURES

- Figure 1: shows the wound healing effect of CP secreted proteins on a compromised fibroblast monolayer in vitro;
- Figure 2: shows the wound healing effect of CP secreted proteins on a compromised keratinocyte monolayer in vitro;
- Figure 3: shows the wound healing effect of CP conditioned media on an in vitro fibroblast wound model in vitro;
- Figure 4a: shows the amount of VEGF produced by the choroid plexus of donor pigs;
- Figure 4b: shows the estimated minimum daily therapeutic daily dose of VEGF produced by donor pigs;
- Figure 5: shows the various manufacturing processes of CP conditioned media to produce a therapeutic composition of the invention;
- Figures 6a-6e: shows the effect of proteins secreted from encapsulated CP cells on fibroblast, smooth muscle cell and inflammatory cell migration (figures 6a and 6d control; figures 6b, 6c and 6e encapsulated CP cells);
- Figure 7: shows the effect of CP conditioned media in reducing the wound area of a chronic wound model in vivo;
- Figure 7a: Part I: shows evidence of healing of a chronic wound using lyophilised CP conditioned media in vivo versus control (lyophilised ENDO-SFM); Part 2: shows wound traces based on the presence of hair follicles to demonstrate evidence of full thickness wound healing;
- Figure 7b: shows a cross section of tissue from the chronic wound of CP conditioned media in treated or non-treated animals at 5x magnification;
- Figure 7c: shows the tissues of figure 6b at 10x magnification;
- Figure 8: shows the wound strength of tissue wounded by simple incision and either treated with CP conditioned media (treated) or with control media (untreated);
- Figure 9a-9d: shows the effect of CP conditioned media on the healing of a chronic wound in vivo in treated (figures 9a, 9b, 9c) and control animals (figure 9b) at low magnification (x4), and between treated and control animals at higher magnification (figure 9d)
- Figure 10: shows a transmission electron microscopy of normal CP clusters cultured in vitro;
- Figure 11: shows a CP capsuloid in vitro produced by culture with media containing matrix proteins;
- Figure 12a: shows the CP capsuloids of figure 9 at low magnification (x10);
- Figure 12b: shows the CP capsuloids of figure 9 at very low magnification (x4);
- Figure 13: shows VEGF secretion in vitro from CP conditioned media extracted from normal CP cultured cells and from cultured CP capsuloids at 7 and 14 days;
- Figures 14a-14b: shows increased protein secretory vesicles in CP capsuloids cultured in ENDO-SFM media (figure 14b) compared to capsuloids cultured in RPMI-CNP media (figure 14a);
- Figure 15: shows the expression level changes of CP capsuloids cultured in ENDO-SFM or ECM composite compared to normal CP cells cultured in RPMI-CPN after 3 days (figure 15a and figure 15b) or 16 days (figure 15c and figure 15d);
- Figure 16: shows the coulter particle analysis of PLGA-CPCM nonspheres;
- Figure 17: shows a scanning electron microscope of PLGA-CPCM nanospheres; and
- Figure 18: shows a schematic drawing of the wound healing process;
- Figure 19: shows HUVEC co-cultured with CP capsuloids (19A and 19B);
- Figure 20: shows HUVEC co-cultured without CP capsuloids.
- Figure 21: shows tissue handling devices: (A) histology fixture containing control tissue; (B) histology fixture containing LCM-treated tissue; (C) explanted skin around incision placed in sampling fixture for tensiometry. Clamping mechanism ensured centering of incision in jig and even tissue sampling. An aluminum block was placed on top of the razor blades, and then a press was used to cut through the tissue.
- Figure 22: shows initial tissue culture of digested choroid plexus clusters. (Top) Clusters were counted over the first 8 days in separate samples each containing 2 CP equivalents. Values shown are mean + S.E.M. (Bottom) Light microscopy of CP clusters in RPMI-CPN tissue culture medium at 2 and 8 days post-isolation. Scale bar = 250 µm.
- Figure 23: shows VEGF output as measured by ELISA in separate collection flasks normalized per CP equivalent, as aliquoted at the initiation of the experiment. Values represent mean +/- S.E.M.
- Figure 24: shows percent reduction of areas in the in vitro scratch assay containing NHEK cells (Top) and NHDF cells (Bottom). Encapsulated CP was separated by Transwell membranes from cultured monolayers at the doses indicated on the x-axis, which represent the number of encapsulated CP clusters per well per mL basal culture medium. Values represent mean percent area reduction ([starting area - final area] / [starting area]) +/- S.E.M.
- Figure 25: shows selected images of open wounds processed for H&E histology. (A) normal rat skin that was never injured, (B) bacitracin-treated open wound, (C) open wound treated with LCM in bacitracin, and (D) open wound treated with DLCM in bacitracin. Wounds were treated for 10 days and sampled 4 days later. Scale bar = 200 µm.
- Figure 26: shows central morphometry of open wounds. Values show mean + S.E.M. (A) Epidermal appendages were counted across the entire central cross sections. (B) Nuclear counts within the central portion mid-way through the wound, and (C) Area of the wound mid-way through the tissue block.
- Figure 27: shows treatment of linear incisions with lyophilized CP conditioned media in bacitracin ointment. Representative macro photos are shown after 8 days of treatment. (A) Control ENDO-SFM, (B) LCM, and (C) DLCM.
- Figure 28: shows treatment of linear incisions with lyophilized CP conditioned media in bacitracin ointment. Representative images following 10 days of treatment and an additional 4 days of healing. (A) Control ENDO-SFM, (B) LCM, and (C) DLCM. Scale bar = 100 µm.
- Figure 29: shows treatment of linear incisions with lyophilized CP conditioned media in bacitracin ointment. Peak break strength in 1-cm wide sections of skin, centered at the incision, as measured by Instron tensiometry.(**p<0.01).

### DEFINITIONS

"Wound" as used throughout the specification and claims means damage to one or more tissues, including open wounds such as cuts, scrapes, surgical incisions and the like, both acute and chronic, as well as internal wounds, for example, bruises, haematomas, fascia or soft tissue damage, hernia, abdominal wall damage, and the like as well as burns. "Wounds" may refer to tissue damage that occurs by trauma, or damage to tissue resulting from pathological sequelae, for example ischemia, that is exhibited due to overlying disease. The present invention is particularly useful in the healing of chronic open wounds such as ulcers, and diabetic ulcers in particular.

"Isolated" or "purified" as used herein refers to secreted CP proteins that have been extracted from the CP conditioned media. Such extraction may include dialysis to remove salts from the media before lyophilising, or the CP conditioned media can be lyophilised directly.

In this disclosure, "comprises," "comprising," "containing" and "having" and the like can have the meaning ascribed to them in U.S. Patent law and can mean " includes," "including," and the like; "consisting essentially of" or "consists essentially" likewise has the meaning ascribed in U.S. Patent law.

### DETAILED DESCRIPTION OF THE INVENTION

The present invention shows for the first time that CP conditioned media comprises a cocktail of proteins that can be used in accelerating wound healing. In particular, this protein cocktail appears to be useful in all of the three characteristic phases of wound healing, i.e. the inflammatory phase, the proliferation phase, and the differentiation phase.

For example, when wounds are treated with CP conditioned media there is an increase in the number of macrophages and an earlier migration of macrophages to the wound site (inflammatory phase), less collagen is deposited (proliferation phase) and there is a significant reduction in scar tissue formation (differentiation phase).

Thus, CP secreted proteins appear to be a powerful regulator of the wound healing process and can be manipulated to prevent scar formation and resulting loss of function that would normally occur in injured tissue during the natural wound healing process. Lack of scarring is also important for cosmetic purposes, especially when the wound affects external portions of the body which are easily seen, such as the face, neck, hands etc.

The choroid plexus (CP) are lobulated structures comprising a single continuous layer of cells derived from the ependymal layer of the cerebral ventricles. One function of the choroid plexus is the secretion of cerebrospinal fluid (CSF). Cerebrospinal fluid fills the four ventricles of the brain and circulates around the spinal cord and over the convexity of the brain. The CSF is continuous with the brain interstitial (extracellular) fluid, and solutes, including macromolecules, are exchanged freely between CSF and interstitial fluid. In addition to the production of CSF, the choroid plexus has been associated with the formation of the CSF-blood barrier⁶. However, its broader function is the establishment and maintenance of baseline levels of the extracellular milleu throughout the brain and spinal cord, in part by secreting a wide range of growth factors into the CSF. Studies have reported the presence of numerous potent trophic factors within choroid plexus including TGFb, GDF-15, GDNF, IGF2, NGF, NT-3, NT-4, BDNF, VEGF, and FGF2^{7,8}

The CP secreted factors have been previously described for use in the treatment of neurological disorders (WO 00/66188) and in the treatment of autoimmune disorders (WO 2006/132548), however, there has been no disclosure or suggestion of CP secreted proteins being useful in the treatment of wounds, and in particular for treating chronic wounds, such as ulcers. In particular, whilst it is known that the CP secretes VEGF⁸, which is known to be involved in angiogenesis, ie in the proliferation stage of wound healing, as the CP also secrets a myriad of additional factors, mainly involved in CSF integrity, it was highly surprising that a composition including all of the CP secreted proteins would be highly effective at improving wound healing, and especially in improving the healing of chronic wounds.

The present disclosure is thus directed to a therapeutic composition comprising purified or isolated CP secreted proteins for use in wound healing. The CP secreted proteins are isolated or purified from CP conditioned media. The therapeutic composition comprises a mixture (e.g., two or more; any combination thereof) of CP secreted proteins including VEGF, CTGF, PDGF, BMP-7, FGF-2, TGF-β, TGF-α, IL8/CXC chemokines, IGF-2, Cystatin, beta-2-microglobulin, Pleiotrophic factor β, PTHLH and other growth factors and extracellular matrix factors.

CP secreted proteins, extracted from CP conditioned media and which are useful in the method of the present invention, may be tested for biological activity in an animal model or in vitro model of wound healing, as discussed below, and suitably active compositions formulated into pharmaceutical compositions. The pharmaceutical compositions of the present invention may comprise, in addition to CP secreted proteins described herein, a pharmaceutically acceptable excipient, carrier, buffer, stabiliser or other material well known in the art. Such materials should be non-toxic and should not interfere with the efficacy of the active ingredient. The precise nature of the carrier or other material will be dependent upon the desired nature of the pharmaceutical composition, and the route of administration e.g. oral, intravenous, cutaneous, subcutaneous, intradermal, topical, nasal, pulmonary, intramuscular or intraperitoneal.

Pharmaceutical compositions for oral administration may be in tablet, lozenge, capsule, powder, granule or liquid form. A tablet or other solid oral dosage form will usually include a solid carrier such as gelatine, starch, mannitol, crystalline cellulose, or other inert materials generally used in pharmaceutical manufacture. Similarly, liquid pharmaceutical compositions such as a syrup or emulsion, will generally include a liquid carrier such as water, petroleum, animal or vegetable oils, mineral oil or synthetic oil.

For intravenous, cutaneous, subcutaneous, intradermal, intramuscular, intraosseous, intraocular, or intraperitoneal injection, the active ingredient will be in the form of a parenterally acceptable solution which is pyrogen-free and has suitable pH, isotonicity and stability.

For topical administration, the active ingredient will be dissolved or suspended in a suitable emollient and may be formulated in the form of a cream, roll-on, lotion, stick, spray, ointment, paste, or gel, and can be applied directly to the wound site or via a intermediary such as a pad, patch or the like.

For nasal or pulmonary administration, the active ingredients will be in the form of a fine powder or a solution or suspension suitable for inhalation. Alternatively, the active ingredients may be in a form suitable for direct application to the nasal mucosa such as an ointment or cream, nasal spray, nasal drops or an aerosol.

Therapeutic compositions can include isolated or purified choroid plexus (CP) secreted proteins (e.g., two or more of those delineated herein), and a clinically acceptable superficial wound treatment article (e.g., negative pressure therapy devices, bandages, films, adhesives, and the like). Such devices, bandages, films, adhesives, and the like are known in the art, and include for example, Also within the invention is a patch to deliver active chemotherapeutic combinations herein. A patch includes a material layer (e.g., polymeric, cloth, gauze, bandage) and the compound of the formulae herein as delineated herein. One side of the material layer can have a protective layer adhered to it to resist passage of the compounds or compositions. The patch can additionally include an adhesive to hold the patch in place on a subject. An adhesive is a composition, including those of either natural or synthetic origin, that when contacted with the skin of a subject, temporarily adheres to the skin. It can be water resistant. The adhesive can be placed on the patch to hold it in contact with the skin of the subject for an extended period of time. The adhesive can be made of a tackiness, or adhesive strength, such that it holds the device in place subject to incidental contract, however, upon an affirmative act (e.g., ripping, peeling, or other intentional removal) the adhesive gives way to the external pressure placed on the device or the adhesive itself, and allows for breaking of the adhesion contact. The adhesive can be pressure sensitive, that is, it can allow for positioning of the adhesive (and the device to be adhered to the skin) against the skin by the application of pressure (e.g., pushing, rubbing,) on the adhesive or device.

In a further instance, the disclosure contemplates the use of one or more additional compounds co-administered with the pharmaceutical composition of the present invention to give an additive or synergistic effect to the treatment regime. Such compounds will generally be substances aimed at enhancing the rate and quality of wound healing. Examples of such substances include glucocorticosteroids, such as prednisone and methylprednisone, nonsteroidal anti-inflammatory drugs (NSAIDS), growth factors (PDGF, EGF, IGF, VEGF, TGF-Beta, and others), antibiotics, trophic or tropic compounds, peptides, mimetics, oligonucleotides, antibodies, inhibitors, as well as first and second generation anti-TNFα agents. Such substances may be administered either separately, sequentially or simultaneously with the CP secreted protein compositions described herein depending upon the type of wound to be treated as will be appreciated by a skilled worker.

The present invention also provides improving tissue wound healing comprising the step of administering an effective amount of the therapeutic composition of the invention to a patient in need thereof. The patient is preferably a human patient, but the method of the present invention may also be used to improve wound healing in non-human animals.

Methods delineated herein include those wherein the subject is identified as in need of a particular stated treatment. Identifying a subject in need of such treatment can be in the judgment of a subject or a health care professional and can be subjective (e.g. opinion) or objective (e.g. measurable by a test or diagnostic method). Methods delineated herein also include those wherein the subject is successfully treated, their condition/symptom ameliorated or their condition/symptom is improved.

Wound healing is improved in a human or animal patient via one or more of the following mechanisms:
a) a decrease in the time of wound recovery;
b) an acceleration and increase in the inflammatory response; and
c) a decrease or inhibition of scar tissue formation,
thereby resulting in improved functionality and cosmetic appearance of the treated tissue.

A particularly preferred application of the compositions comprising CP secreted proteins described herein, is in the treatment of chronic wounds such as diabetic ulcers.

Another preferred application of the present disclosure is in the treatment of skin wounds.

The ability of the compositions of the invention comprising CP secreted proteins to treat superficial or deep skin wounds can be demonstrated according to known methods⁹, and as exemplified in example 5, below.

Another preferred application in the present disclosure is in the treatment of bums. The ability of CP secreted proteins to treat burn wounds can be demonstrated in known animal models. For example as described in Yang *et al*¹⁰*.*

Administration of the pharmaceutical composition of the invention is preferably in a "prophylactically effective amount" or a "therapeutically effective amount", this being sufficient to show benefit to the individual. The actual amount administered, and rate and time-course of administration, will depend on the nature and severity of the type of wound that is being treated. Prescription of treatment, e.g. decisions on dosage etc., is within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in Remington's Pharmaceutical Sciences, 16th edition, Oslo, A. (ed), 1980.

The present disclosure is also directed to the use of CP secreted proteins in the manufacture of a medicament for improving wound healing in a patient in need thereof. The CP secreted proteins may be isolated or purified from CP conditioned media.

The present invention is directed to CP conditioned media for use in improving wound healing in a patient in need thereof.

The medicament may be formulated for local or systemic administration, for example, the medicament may be formulated for topical administration to an external or open wound site, or may be formulated for injection into an internal or deep wound site, as described above.

The medicament may further comprise one or more additional immuno-responsive compounds to give an additive or synergistic effect on wound healing, selected from the group of immuno-responsive compounds described above. The medicament may be formulated for separate, sequential or simultaneous administration of the CP secreted proteins/CP conditioned media and the one or more immuno-responsive compounds.

The present disclosure is also directed to a method of manufacturing the therapeutic compositions comprising CP secreted proteins, said method comprising the steps:
a) culturing CP cells in vitro;
b) extracting CP conditioned media;
c) lyophilizing the CP conditioned media; and
d) optionally dialysing CP conditioned media, either before or after step c)

The CP cells may comprise a purified population of CP cells or they may alternatively comprise CP-derived cells selected from the group comprising glial or glial-derived cells, epithelial cells, multipotent neuronal precursor cells, proginator cells and cells positive for neuronal precursor cell markers (such as Neu-N).

The CP cells may be obtained directly from a suitable mammal donor or may be obtained from primary or secondary CP cell culture or from a CP cell line including immortalised CP cell lines, or from a combination of any of the above sources. The CP cells in culture may have been genetically modified to produce one or more desired proteins useful in promoting wound healing, using genetic modification techniques well known in the art. The CP cells obtained directly from a donor, may comprise cerebro spinal fluid containing one or more CP cells. Suitable CP cell donors include pigs, sheep, cows, goats, rabbits, mice and primates including rhesus monkeys and humans. The CP, cells may be isolated from donors using known methods in the art, for example, as described in WO 00/66188.

The CP cells may comprise isolated cells or clusters of cells and may be "naked" or encapsulated, for example, in alginate. Where CP cells are "naked", they may be "free" to make direct contact with one another or they may be separated by a biocompatible separation means which allows the diffusion of secreted proteins from the CP cells. The encapsulation of the CP cells may function as such a biocompatible separation means. CP cells may be encapsulated using methods known in the art (see for example WO 00/66188 and US 6322804).

The CP cells are cultured in suitable culture media for a period of time and under conditions that allow secretion of the numerous secretion proteins including growth factors etc, from the CP cells into the media. The conditioned media is then separated from the cells to provide a CP secretory protein rich, cell-free CP preparation.

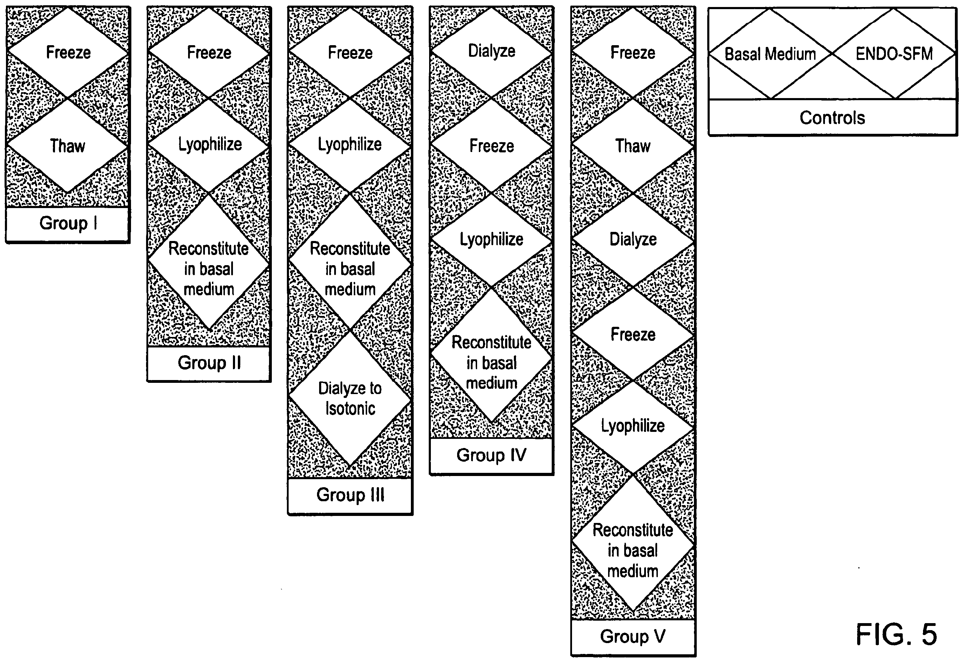

The CP conditioned media can be prepared by a series of known steps to produce a therapeutic composition, including the optional step of dialysis (to remove salts), before or after lyophilisation, as set out in figure 5. The processing steps resulted in a 6-9 times concentration of VEGF in the therapeutic composition, as described in example 4. It is also expected that other CP secretory proteins useful in promoting wound healing would also be concentrated in the lyophilised CP conditioned media.

The lyophilised CP conditioned media may be combined with a pharmaceutically acceptable excipient and used in the methods of the invention to promote wound healing, as described above.

The present invention provides a method of manipulating the profile of the proteins secreted by CP cells in vitro, said method comprising the steps:
a) culturing CP cells in vitro in suitable basal growth media;
b) adding media containing matrix proteins in an amount sufficient to initiate the formation of CP capsuloids,
wherein the CP capsuloids comprise a significantly higher level of protein secretory vesicles when compared to CP cells grown in basal growth media alone (e.g., without further steps, without further additional agents, without step (b)).

The media containing matrix proteins is a media rich in collagen IV, laminin and other ECM components and results in the formation of CP capsuloids, as described in example 6.

Capsuloid formation results in an increase in the number of protein secretory vesicles and a corresponding increase in the level of secreted proteins, particular VEGF. This increase in protein secretion is the result of increased protein expression brought about by the media composition and capsuloid formation. It is contemplated in the present invention that the protein cocktail secreted by the CP capsuloids can be manipulated to maximise the therapeutic potential.

Without being bound by theory, it is thought that CP secreted proteins are effective in improving wound healing by acting at all three recognised phases of wound healing, i.e. the inflammatory phase, the proliferation phase and the differentiation phase described above. Whilst the exact mechanism is unknown, it is likely a result of the combination of growth factors secreted by the CP, some of which have been shown previously to have been involved in wound healing. Such factors include VEGF, CTGF, PDGF, BMP-7, FGF-2, TGF-β, TGF-α, IL8/CXC chemokines, IGF-2, Cystatin, beta-2-microglobulin, Pleiotrophic factor β, PTHLH and other growth factors and extracellular matrix factors. Such a combination has not previously been used in wound healing. In addition, a number of other factors secreted by CP, such as neurotrophin (NT) factors and transthyretin are not known to be actively involved in wound healing and yet other factors, such as retinoic acid, secreted by the CP are known to inhibit wound healing (US 5973007).

It is therefore surprising that a cocktail of CP secreted proteins (e.g., comprising those delineated in Tables 4 and 5), including retinoic acid and possibly other proteins that do not promote wound healing, are extremely effective in improving wound healing, and in particular, in enhancing the healing of chronic wounds.

For example, CP secreted proteins in CP conditioned media has a direct effect on macrophage recruitment. In particular, both the number of macrophages and, presumably, the migration time to the wound site are increased when animals are treated with CP secreted proteins (results not shown). Thus, the first phase of wound healing, the inflammatory phase, is significantly improved and it is expected that this will result in faster and more efficient wound cleansing and angiogenesis.

In addition, CP secreted proteins in CP conditioned media also result in less collagen being deposited in the proliferation phase (results not shown). The protein cocktail secreted by the CP is thought to be a chemo-attractant for inflammatory cells, fibroblasts, endothelial cells, and epithelial cells,. Thus, use of CP secreted proteins is thought to result in a proportionate decrease in the relative recruitment of fibroblasts to the wound site and thus less overall production of collagen by the reduced population of fibroblasts.

CP secreted proteins are further involved in inhibiting scar tissue formation in the differentiation phase of wound healing. Specifically, use of CP secreted proteins results in a significant reduction in scar tissue formation in a recovered wounded tissue. In addition, there was also a significant reduction in loss of functional tissue, i.e. use of CP secreted proteins also resulted in improved tissue regeneration, so that the recovered tissue was replaced without scarring and thus had little functional or cosmetic impairment. This may be particularly beneficial in cosmetic surgery or in treating wounds to portions of the body that are clearly visible, such as face, neck, hands etc.

It is expected that the present invention will be useful to treat all types of wounds such as skin cuts and abrasions, deep wounds extending through the skin and muscle (including surgical incisions) as well as internal wounds (for example wounds to muscle and tendon caused by sports injury or trauma), bruises, hematomas, , fascia, soft tissue damage, hernia, abdominal wall damage, as well as bums and chronic wounds such as ulcers, in particular, diabetic ulcers which are very difficult to treat using conventional therapy.

The described microarray was used to investigate the transcriptome of porcine CP epithelium, and then assess the in vitro and in vivo regenerative capability of secreted CP products in cell monolayers and full thickness cutaneous wounds. In vitro, CP reduced the void area of fibroblast and keratinocyte scratch cultures by 70% and 33%, respectively, compared to empty capsule controls, which reduced the area by only 35% and 6% respectively. In vivo, after 10 days of topical application, CP conditioned medium lyophilate dispersed in antibiotic ointment produced a 2-fold improvement in incision tensile strength compared to ointment alone, and an increase in the regeneration of epidermal appendages from roughly 50 to 150 features per wound. Together, these data identify the CP as a source of secreted regenerative molecules to accelerate and improve the healing of topical wounds and potentially other similar indications. Based on the abundance and significance of its components within the wound healing process, the simultaneous application of the entire CP cocktail would provide relevant molecules "as-needed," avoiding having to orchestrate delivery with the precisely staged action of cells, proteins, and matrix elements that occurs in the natural healing cascade. In vitro, hydrogel encapsulated porcine CP was co-cultured with scratched monolayers of fibroblast or keratinocytes to assess migration and proliferation triggered by CP molecules. In vivo, we used CP conditioned media lyophilate to treat full thickness open wounds and sutured skin incisions. Additionally, we collected mRNA from CP used to generate conditioned media to identify genes associated with potential regenerative factors expressed in this specific culture system.

### EXAMPLES

### Materials and Methods

### Choroid Plexus Isolation

Neonatal Yorkshire pigs aged 7-10 days were induced with ketamine (500 mg/kg) and xylazine (0.15mg/kg), and then maintained on 3% isoflurane during controlled exsanguination. Following craniotomy, the brain was removed and hemisected along the midline to expose the CP in the lateral ventricles. The CP were extricated and placed in Hanks Balanced Salt Solution (HBSS, 0-4°C) supplemented with ciproflaxocin (Sigma, USA, 3µg/ml), 2% human serum albumin and digested as previously described (Borlongan, C. V.; et al. Neuroprotection by encapsulated choroid plexus in a rodent model of Huntington's disease. Neuroreport 15:2521-2525; 2004; Emerich, D. F. et al., Extensive neuroprotection by choroid plexus transplants in excitotoxin lesioned monkeys. Neurobiol. Dis. 23:471-480; 2006). Briefly, following mechanical disruption with scissors, CP epithelial clusters were released after light enzymatic digestion over 20 minutes (Liberase HI, Roche, New Jersey). Epithelial clusters were washed and maintained in RPMI-1640 (Sigma, St. Louis, MO) with 2% neonatal porcine serum and 3 µg/mL ciproflaxocin (RPMI-CPN) and placed in non-adherent flasks at 37°C with 5% CO₂. Half of the media was removed and replaced with fresh media after 24 hours, 48 hours and every other day thereafter. After 0, 2 and 8 days in RPMI, clusters were counted and separated into individual flasks. After 10 days, the media was replaced with serum-free endothelial cell culture medium (ENDO-SFM, Gibco, USA), which was used throughout the experiment for generation of conditioned media over intervals of 24, 72 or 96 hours as previously described (Thanos, C. G.; et al., The in vitro expression and secretion of vascular endothelial growth factor from free and alginate-polyornithine encapsulated choroid plexus epithelium. Tissue Eng. 13:747-756; 2007).

### Isolation of RNA and Gene Array

RNA was isolated from cultured CP clusters after 3 or 16 days in ENDO-SFM tissue culture medium (N=3). Cell clusters were disrupted and homogenized in buffer RLT using a QIAshredder (Qiagen, USA). Briefly, 700 µl of cell lysate was loaded onto the QIAshredder, centrifuged at 30,000 x g, and RNA was purified using an RNeasy Mini spin column (Qiagen, USA). Purified, high-quality RNA was eluted in RNase-free water (Gibco, USA). Single-stranded cDNA was synthesized by priming with a T7 Oligo(dT) primer, and then converted into double-stranded DNA templates for transcription by employing DNA polymerase and RNase H for simultaneous degradation of RNA and synthesis of the second cDNA strand. The cDNA was purified and cRNA was subsequently synthesized and amplified using in vitro transcription. After final purification of the cRNA, sample integrity was confirmed on electropherograms acquired from a Bioanalyzer 2100 (Agilent Technologies, USA). Once samples were confirmed to be suitable for array analysis, a whole-genome porcine microarray (Affymetrix, USA) was used to characterize the entire pig genome. Genes with log₂ expression levels < 7.5 were excluded from the analysis. Genes with relevance to the wound healing process were included in Table 5 based on their relative magnitude of expression and statistical significance (independent t-test, p<0.05). Genes that were differentially expressed over time were included in Table 6 based on the magnitude of change (> +/- 1.85-fold) and statistical significance (independent t-test, p<0.05).

### In Vitro Bioactivity: Scratch Assay

CP clusters were encapsulated in alginate-polyomithine-alginate (APA) microcapsules after 10 days in culture as previously described at 50,000 clusters/mL using a coaxial air jet system (Emerich, D. F.; et al. Extensive neuroprotection by choroid plexus transplants in excitotoxin lesioned monkeys. Neurobiol. Dis. 23:471-480; 2006) 600 µm diameter capsules were produced and were incubated in RPMI-CPN for 5 days prior to transfer to fibroblast or keratinocyte cultures. A sample was pulsed in fresh RPMI-CPN for 24 hours to quantify secreted VEGF. Normal human dermal fibroblasts (NHDF, Cambrex, USA) or normal human epidermal keratinocytes (NHEK, Cambrex, USA) were expanded in T75 flasks in supplemented cell culture medium. NHEK cells were grown in Keratinocyte Growth Medium (KGM, Cambrex, USA) supplemented with epidermal growth factor, insulin, and hydrocortisone. NHDF cells were growth in Fibroblast Growth Medium (FGM, Cambrex, USA) supplemented hFGF-B and insulin. After several passages, cells were seeded into 24-well plates at 5,000 cells/well (N=8 per treatment group), and grown to 95% confluency. To initiate the scratch assay, the culture medium was removed, the cells were washed with HBSS three times, and a defect (approximately 9 mm²) was created with the tip of a 20 µL pipette. The HBSS was aspirated several times to loosen the cells surrounding the defect, and then replaced with basal culture medium. The defects were then photographed at 4X magnification. Transwell inserts with a pore size of 10 µm were placed on top of the wells, and encapsulated CP were added for an equivalent dose of 500, 1500, 2500, or 5000 CP clusters per well. Empty capsules served as controls and were also incubated in RPMI-CPN for 5 days, and then added to wells at the peak dose range of the encapsulated cell group, corresponding to a 100-µL volume of capsules. Cultures were allowed to incubate for 7 hours (NHEK) or 14 hours (NHDF), the capsules and inserts were removed, and the defects were photographed again at 4X magnification. Calibrated image analysis software (Motic, China) was used to measure the scratched area prior to and following exposure to encapsulated CP.

### Collection and Preparation of CP Proteins for Topical Application

CP conditioned medium (CM) was collected at 96 hour intervals in ENDO-SFM from flasks containing roughly 100,000 CP clusters in 28 mL medium. Following the initial culture period in RPMI-CPN and subsequent transition to ENDO-SFM, CM was collected repeatedly for a period of 2 months. Briefly, cell clusters were allowed to sediment by gravity, and then conditioned media was aspirated and replaced with fresh ENDO-SFM warmed to 37°C. The CM was collected in 50-mL tubes and immediately flash-frozen in liquid nitrogen. In some cases, the CM was dialyzed against distilled water in a 1000 Da MWCO membrane to remove salts prior to freezing. An aliquot was also taken prior to processing for measuring VEGF content. Frozen CM was lyophilized for 96 hours (Virtis, USA) and stored at -80°C prior to use. Fresh medium was processed in parallel for use in control animals.

Lyophilized CM (LCM), or dialyzed lyophilized CM (DLCM) was prepared for topical administration immediately prior to application by mixing the lyophilate in bacitracin ointment (Johnson & Johnson, USA) at roughly 50 mg LCM / 350 mg ointment for the high dose, and roughly 17 mg LCM / 350 mg for the low dose. The actual amounts were calculated based on the amount of VEGF measured during the media collection period, with the final doses normalized to a VEGF dose of 6 ng at the low dose, and 20 ng at the high dose.

### Detection of Vascular Endothelial Growth Factor (VEGF) using ELISA

VEGF was measured in culture supernatants as a surrogate marker of potency as previously described (Thanos, C. G.; et al., The in vitro expression and secretion of vascular endothelial growth factor from free and alginate-polyornithine encapsulated choroid plexus epithelium. Tissue Eng. 13:747-756; 2007). After 96 hours of incubation (37°C, 5% CO₂) the supernatant was removed and frozen for subsequent analysis. Thawed samples were analyzed in triplicate using a human VEGF ELISA (Quantikine^{®}, R&D Systems, USA). Sequence homology between porcine and human VEGF is greater than 90% (24). Samples of conditioned media (200 µL) were combined with assay diluent (50µL) and the optical density was measured at 450 nm on a Beckman Coulter DTX-880 spectrophotometer after development. The value obtained was compared to a standard curve of recombinant human VEGF using a logarithmic curve fit. Values were expressed as pg/mL.

### In Vivo Evaluation of CPCM Formulations

CPCM was evaluated in full-thickness wounds in male Long-Evans rats approximately 3 months old and weighing between 250-350 g housed in pairs in a temperature controlled environment with a 12:12 hour light-dark cycle. All study groups consisted of 8 animals. All procedures met or exceeded NIH guidelines and were approved in advance by the Brown University IACUC governing body.

Immediately prior to surgery, rats were anesthetized with isoflurane (3-4%) and placed in a nose cone in a prone position. Prior to the creation of the skin defect, animals were clipped and the skin around the area was disinfected with betadine. Following the surgery, animals were treated with an analgesic (buprenorphine, 0.02 mg/kg subcutaneous) and observed until fully recovered from anesthesia. Animals were euthanized using carbon dioxide gas, and explanted tissue was removed as indicated for histological processing. Briefly, after fixation in isotonic 4% paraformaldehyde for 2-3 days, tissue segments were transversely embedded in paraffin for cutting 4 µm-thick sections, which were subsequently stained with either hematoxylin and eosin (open wound tissue), or Masson's trichrome stain (incision wounds). Morphometry on healing wounds was carried out using image analysis software (Motic, China) to measure the healing area within the margin of hair growth, and statistics were calculated with Prism software (GraphPad, USA), using a one-way ANOVA with a 95% confidence interval and post-hoc Dunnet's test for comparison against the control group.

### (Open Wound)

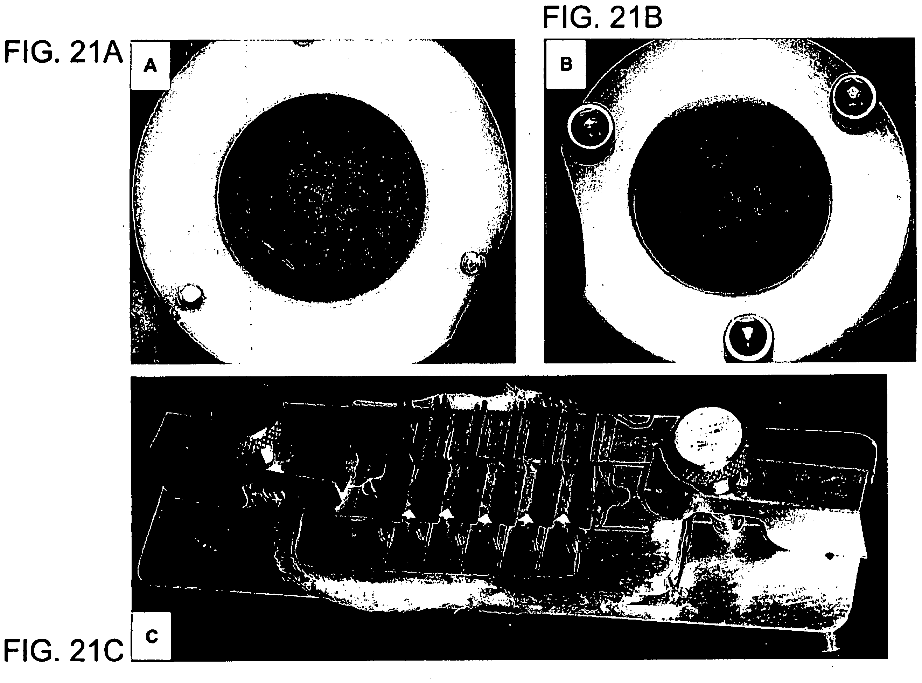

An 8-mm Keyes dermal punch was used to completely remove patches of skin to create full-thickness skin defects to the depth of subcutaneous tissue between the shoulder blades. Topical CM ointment was applied for 10 days following the procedure, and animals were sacrificed at day 14 for tissue harvesting. A control group (lyophilized ENDO-SFM in bacitracin ointment, N=5) and 2 treated groups (high dose LCM in bacitracin ointment, N=7, and high dose DLCM in bacitracin ointment, N=7) were observed. At the conclusion of the study, following euthanasia with CO₂ gas, large sections of skin containing the wounded area were removed and centered in devices consisting of 2 plastic rings held under tension by a series of peripherally located screws. The devices were created to overcome histological artifact associated with skin deformation during the fixation process (Figures 21A and 21B). The skin was kept relaxed during insertion into the devices, preventing additional artifact caused by tension in the wound. Devices with skin samples were immersion fixated in isotonic 4% paraformaldehyde overnight and were subsequently processed for histology and morphometry, which included measurement of cross-sectional wound area, nuclear counts within the central 2mm² area, and counts of epidermal appendages within the entire wound area.

### (Incision)

A 6 cm linear full thickness incision was made between the shoulder blades with a fresh size 15 scalpel through a plastic template designed for uniformity of straightness and length. The incision was immediately closed with 8 interrupted stitches using 6-0 silk equipped with a PC-3 cutting needle. Based on prior experience within our group, silk was chosen to minimize interference in the healing of the wounds caused by irritancy of other bristle-like materials. For the next 10 days, wound sites were treated topically topically once daily in the following groups: a control group (lyophilized ENDO-SFM in bacitracin ointment, N=5) and 2 treated groups (LCM in bacitracin ointment, N=5, and DLCM in bacitracin ointment, N=5). After 14 days, the entire 6 cm wound strip was removed following euthanasia with carbon dioxide gas. After shaving the area and removing the sutures, an area of 7 x 3 cm was excised around the wound and processed for histology and tensiometry. Because pilot studies demonstrated inter-sample variability related to skin harvesting, a specialized instrument was engineered to create uniform 1-cm width strips of skin centered horizontally at the incision with fresh razor blades (Figure 21c). A total of 5 strips of tissue were created per wound size by excluding the outer 0.5 cm on each side. The area of skin was placed onto slabs of dental wax that were visually centered onto the jig, and 6 fresh razorblades were inserted into the positions shown in the picture. A press was used to exert force on an aluminum block resting on top of the blades, creating an evenly distributed force completely perpendicular to the plane of tissue, and resulting in precise 1-cm wide tissue segments for histological and physical analysis. For each animal, the central 1-cm segment was processed for histology, while the outer 2 on each side were analyzed for tensile strength at break using an Instron tensiometer (Instron, USA). Skin samples were immediately transported on ice on their wax backing material to the instrument. Samples were removed from the wax, any residual panniculus camosus was moved from the underside of the wound, and the tissue was placed in the serrated jaws of the instrument under maximum clamping pressure. With a 50N load cell in place, the tissue was pulled at a rate of 8 mm/ min until breakage occurred. Statistics were calculated using Prism software (GraphPad, USA), using a one-way ANOVA with a 95% confidence interval and post-hoc Dunnet's comparison.

### Example 1: Characterisation of the in vitro wound healing potential of choroid plexus conditions medium in suitable wound models

Neonatal porcine choroid plexus, isolated from Yorkshire swine, was isolated and maintained in culture as previously described in Thanos et al⁸. After 7-10 days, choroid plexus clusters were encapsulated in alginate and held for another 7 days.

Monolayers of cell types relevant to wound healing were created in 6-well plates using standard tissue culture techniques to create previously described in vitro wound models. Adult human dermal fibroblasts or kertinocytes were grown to 95% confluency, whereupon the monolayer was physically disrupted with a specialised tool to create a defect on the order or 10-20 mm². Following the creation of the defect, cultures were washed with HBSS, photos were taken for image analysis, and the appropriate basal medium was returned to the plate. In the first experiments, transwells were used to suspend CP-containing capsules (500-5000 eCP equivalents/well) so that the respective cell type would have direct access to secreted proteins. Later, CP conditioned media was collected separately and added at various concentrations along with other media types in an attempt to titrate the effect. After the proper exposure time, treatment was removed and photos were again taken for image analysis. Wound area was measured using Motic software, and % healing was calculated by (areaᵢₙᵢₜᵢₐₗ - areaᵢₙᵢₜᵢₐₗ).

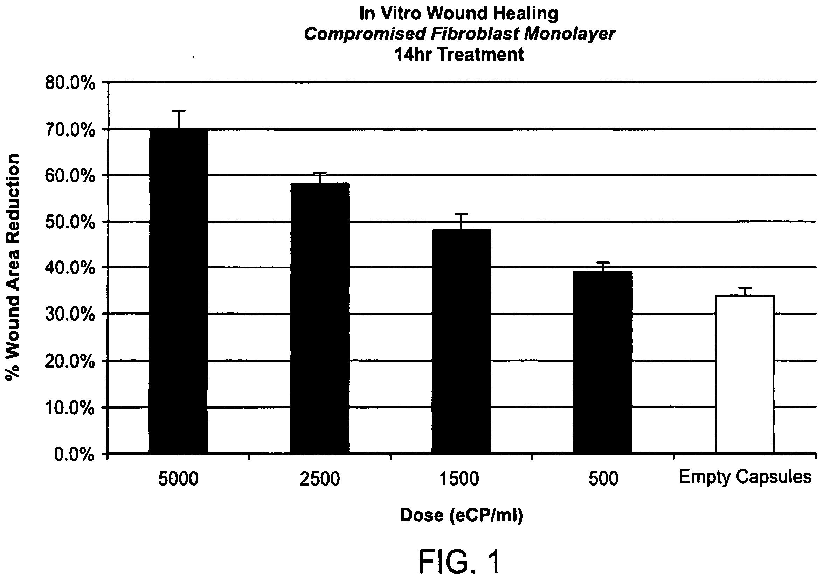

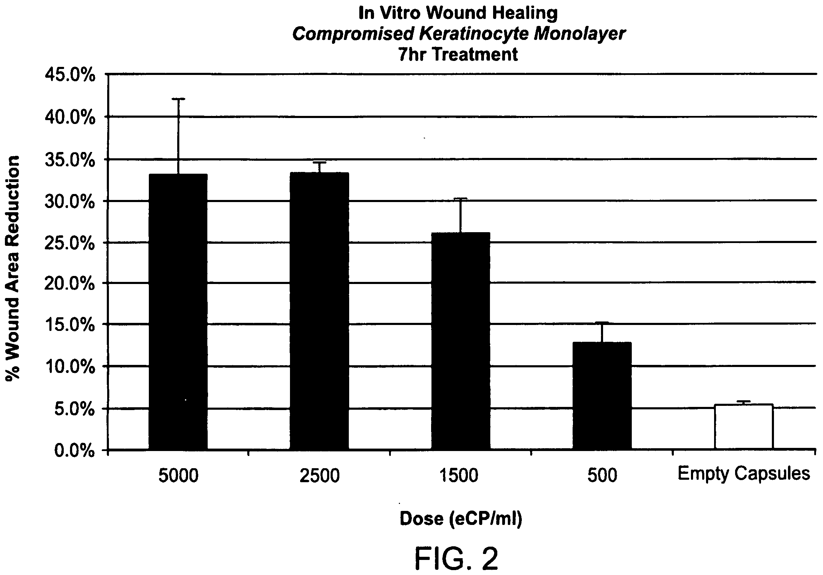

Encapsulated choroid plexus, when separated by a transwell membrane insert, showed a dose dependent increase in healing of both fibroblast (figure 1) and keratinocyte (figure 2) compromised monolayers. The keratinocyte model revealed that CP at a dose of 2500 eCP/mL improved healing relative to the empty capsule control by almost 30%.

### Example 2: Effect of CP conditioned media in vitro

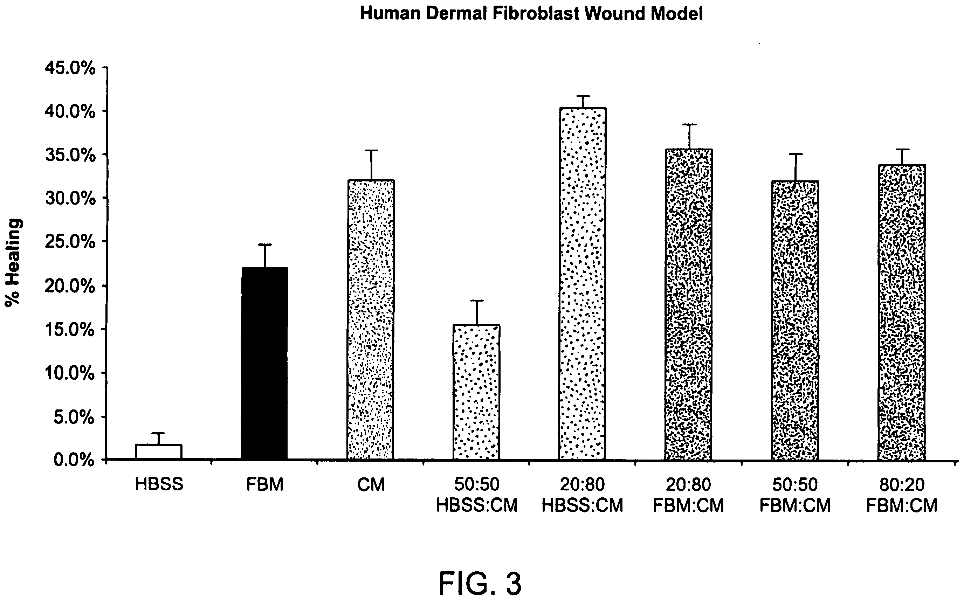

CP conditioned medium was collected for 96 hours after 7 days of culture of CP cell culture and then added to fibroblast culture medium. The therapeutic effect of CP conditioned media and fibroblast wound model is shown in figure 3. Each group containing conditioned media (CM) along with nutrients showed an improvement compared to the basal medium control. Significantly, CM is likely depleted of essential nutrients required to maintain healthy cell cultures, especially compared to a fresh fibroblast basal growth medium (FBM). Still, CM provided benefit in each condition, particularly those that supplement a minimal amount of nutrients.

### Example 3: Assessment and optimisation of the long-term production of VEGF (indicator of wound healing potential) in cultures with daily or 2/week media changes

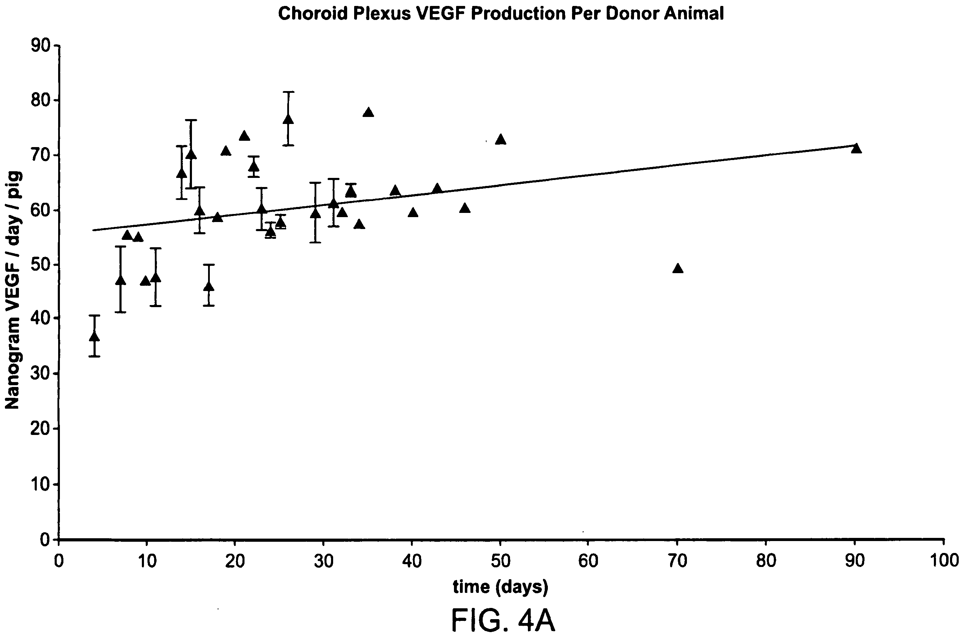

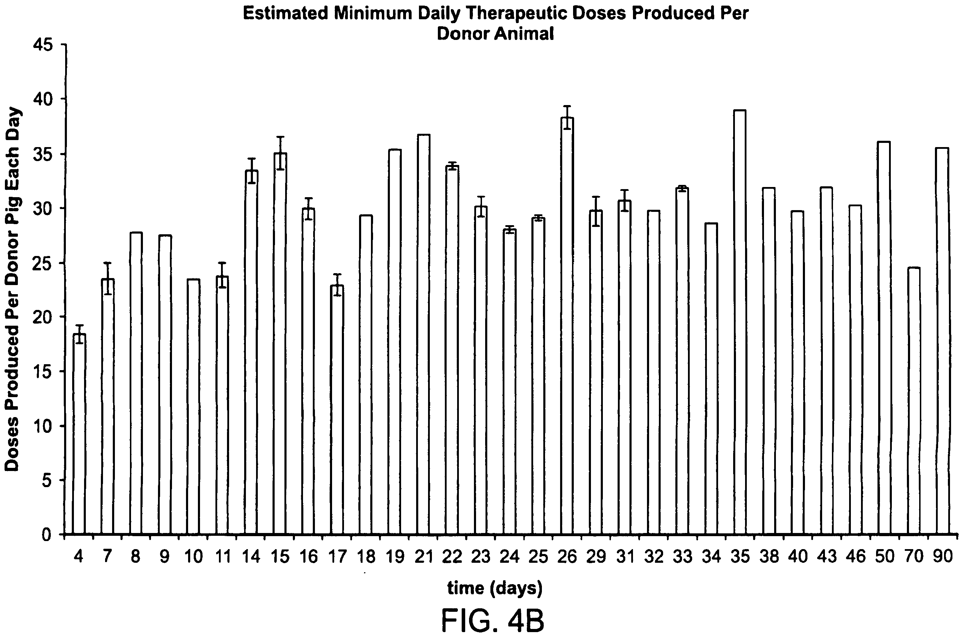

Based on previous work demonstrating that the secretion of VEGF by CP was enhanced in serum-free endothelial growth media⁸, CP clusters were maintained in ENDO-SFM culture for up to 3 months for the continuous collection of therapeutic media for wound studies. In this process, an aliquot of most media collections was kept for VEGF analysis by ELISA. The results, shown in Figure 4a demonstrate the long-term stability of VEGF secretion. Based on a minimum therapeutic dose of 2 ng/day, the equivalent dose production potential is shown in Figure 4b, averaging about 25 doses produced each day by each donor pig CP. Importantly, this signifies that the issue of sourcing is exponentially reduced by choroid plexus secretory factors due to their robustness and stability in long-term cultures.

### Example 4: Preparation of a therapeutic composition of CP secreted proteins

CP conditioned medium was formulated for therapeutic use by either dialyzing out salts and lyophilizing, or lyophilizing directly. The extracted CP proteins were subjected to processing and analysed for VEGF using ELISA. The following specific processing groups tested are set out in figure 5.

### Each final product was reconstituted at 10.8 mg/mL and analysed with the VEGF ELISA:

| Group | Osmolarity (mOsM) | [VEGF] (ng/mL) |
|---|---|---|
| I | 306 | 722 |
| II | 484 | 4,659 |
| III | 319 | 6,310 |
| IV | 311 | 6,860 |
| V | 316 | 5,381 |
| Controls | 287 | ND |

As demonstrated here, processing of conditioned media was not associated with any significant drop in VEGF level. Group I, essentially neat conditioned medium, was concentrated roughly 6-9X in each of the other groups, II-V.

### Example 5: The effect of choroid plexus proteins secreted from encapsulated CP cells on macrophage, collagen, smooth muscle cells and endothelial cell migration in vivo.

Encapsulated choroid plexus clusters (50K / mL) or empty capsules were loaded into Matrigel HC (BD Biosciences), diluted by 50% with saline, at 0.1:1.0 capsules:matrigel for a total injection volume of 1.1 mL. The injectate was kept on ice prior to injection, which occurred through a 16-gauge needle into the subcutaneous space between the shoulder blades of long-evans rats (Male, 250-300 g) under isolflurane anesthesia at 2-3%. 2 weeks later, subcutaneous gel slabs were explanted, fixed in 4% paraformaldehyde, and processed for H&E histology.

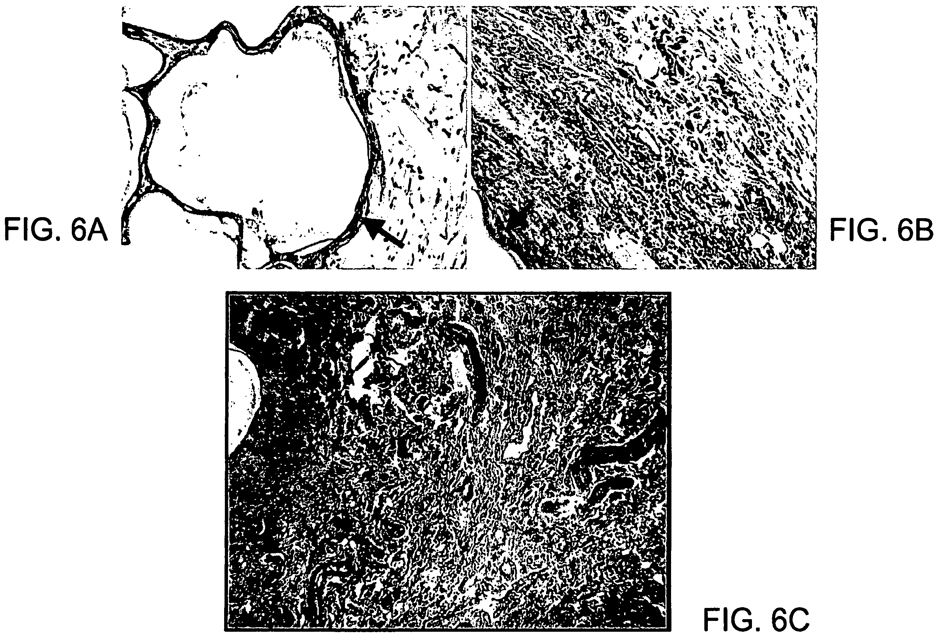

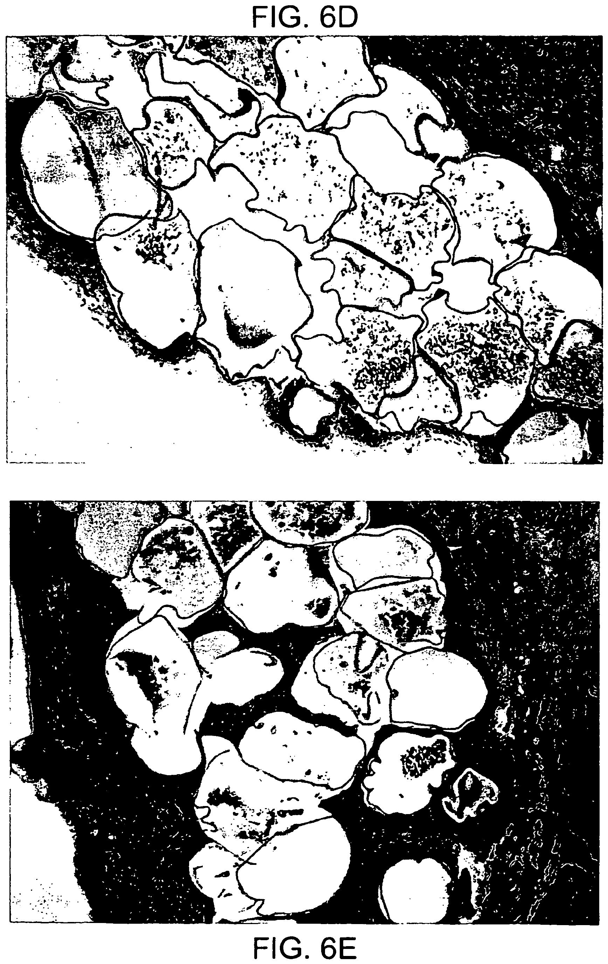

In general, explants containing encapsulated choroid plexus were associated with increased angiogenesis, collagen removal, and structural organization of the surrounding tissue. This included remodeling as shown by the near complete destruction of the matrigel matrix. In comparison, empty capsule implants showed very minor fibroblast infiltration, with fully intact matrigel matrix and little evidence of angiogenesis or chemotaxis. The contrast between the two groups is clear in Figure 6, where an empty capsule implant (Figures 6A and 6D) is shown next to an implant containing encapsulated choroid plexus (Figures 6B and 6E). The granular eosinic material in Figure 6A is the matrigel vehicle, which is largely absent in the encapsulated CP group (Figure 6B). Also apparent is the minimal cellular infiltration in the empty control group compared to encapsulated CP, which apparently caused migration of fibroblasts, macrophages, endothelial cells, smooth muscle cells, and other regenerative cell types to the area. Figure 6C, a high magnification view of the encapsulated CP group, shows further evidence of enhanced angiogenesis around the periphery of the capsule with loss of implanted matrigel and stratified cellular organization.

### Example 6: The effect of concentrated or formulated choroid plexus proteins in vivo, in two animal models of full-thickness wounds

Choroid plexus conditioned media was collected as described in example 2, above, and lyophilized as described in group II of figure 5, discussed in example 4, above. The lyophilised CP conditioned media was reconstituted in distilled water for treatment of full thickness wounds or dispersed in hydrophobic ointment for topical application in 6 cm incision wounds.

In long-evans rats, a type of full thickness chronic wound via skin autograft was created using an 8-mm biopsy punch down to the level of subcutaneous tissue. 2 groups containing 10 animals each was used containing a treatment (lyophilised CP conditioned media) and control (lyophilised ENDO-SFM). On each animal, bilateral defects were made, and the excised skin was transplanted to the contralateral side to assess viability of engrafted hair as well as to provide a biologic enclosure for containment of the injection. Due to the black and white spotting pattern of this rat strain, areas selected so that opposing colours were implanted in juxtaposition to optimise detection of surviving hair follicles. 8 interrupted sutres (6-0 silk) were used to hold the flaps in place. 25 mg lyophilised CM was injected daily for 14 days in 200 µL dH₂0 for a total of roughly 6 ng VEGF/day, administered directly to the wound site. Macro photography was used daily to assess and measure wound area. After 4 weeks, animals were euthanized and wound areas were collected for histology.

Separately in the same strain, straight 6-cm incisions were created with fresh size 15 scalpel blades. Incisions were immediately closed with interrupted stitches of 6-0 silk, and animals were allowed to recover in separate cages. Each day for 10 days, 250 mg ointment was mixed with lyophilised CP conditioned medium to produce a viscous pink paste containing roughly 18 ng VEGF. At day 14, animals were euthanized and the area around the 6-cm wound was excised and cut into 5 1-cm wide strips for instron tensile strength measurement.

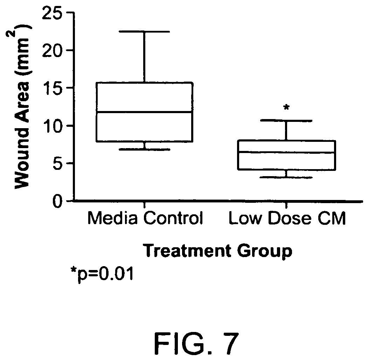

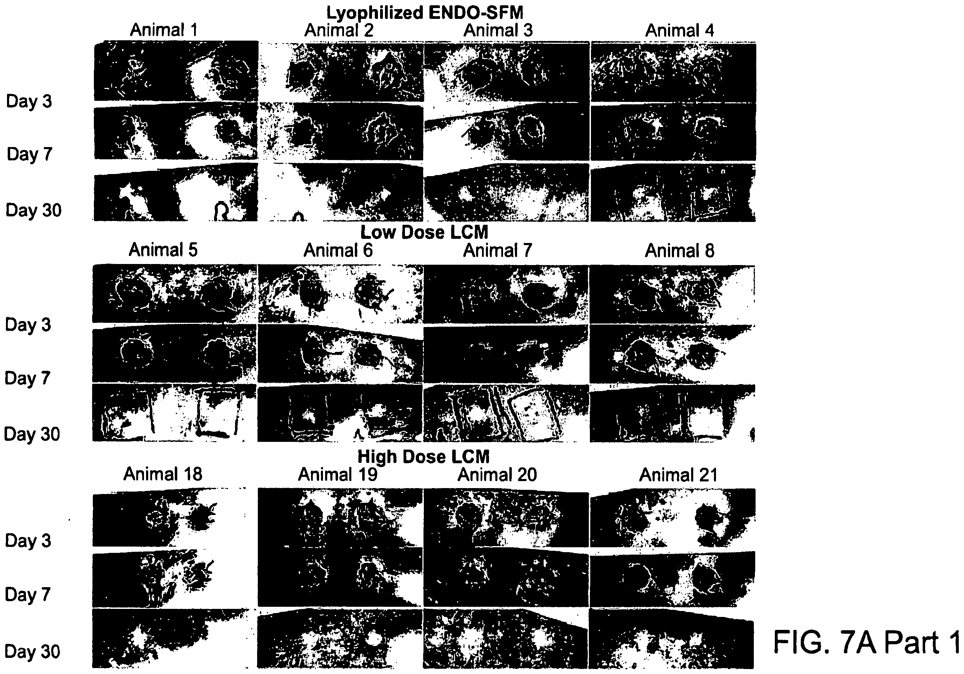

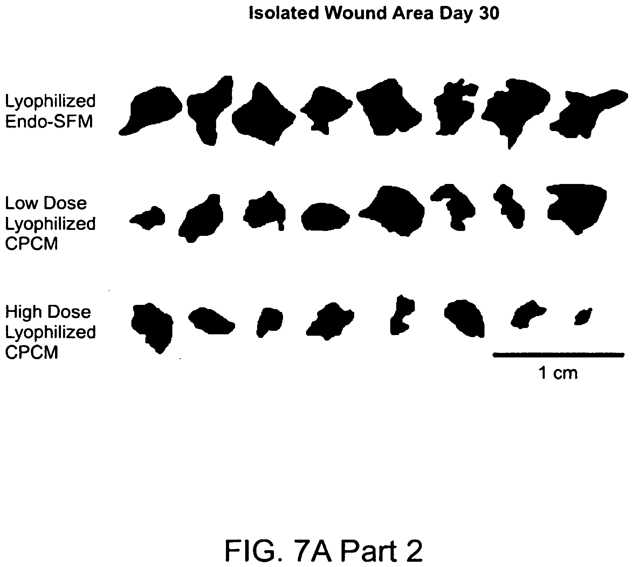

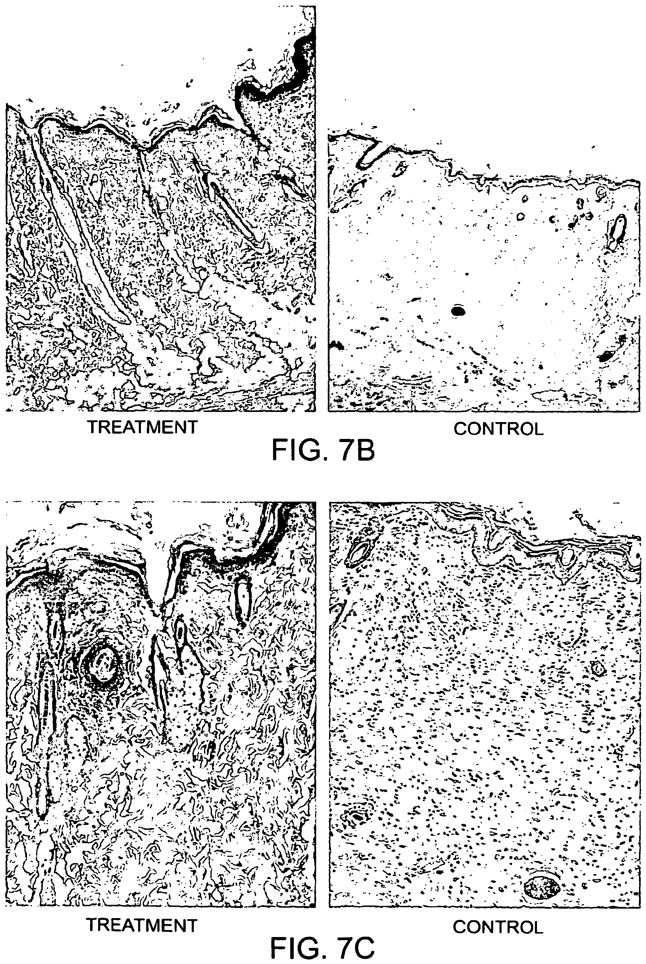

In the chronic wound model, a statistically significant decrease in wound area was observed in the group treated with CP conditioned media on the order of 2.5-fold (figure 7 and figure 7a, part 2). The wounds, shown in figure 7a, part 1, contained areas of counter-coloured hair and were much smaller and healthier in general. The histology, shown in Figures 7b and 7c, shows that a very healthy and organised skin morphology was achieved in the treatment group, whereas the controls (or untreated wound) presented mostly as fibrous masses devoid of neovascularisation with no evidence of normal skin structure. This level of healing is typically associated with much longer durations, on the order of months, while this was achieved within only 30 days with the CP protein treatment.

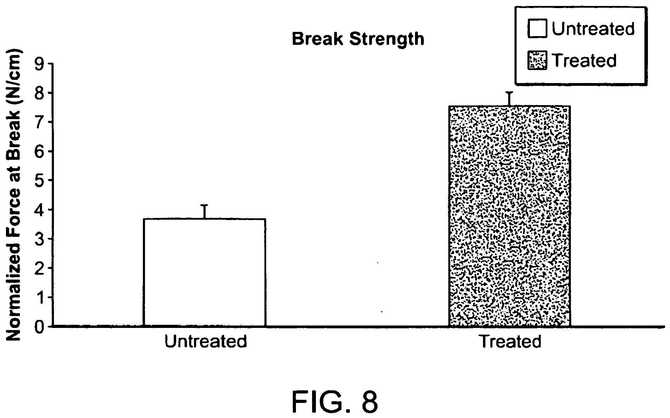

Incisional healing was also greatly accelerated in the presence of CP secreted proteins. In particular, wounds treated showed nearly a 2-fold increase in strength compared to those treated with ointment alone (figure 8). This level of strength improvement and wound acceleration is unique to the CP protein cocktail.

### Example 7: Topical treatment of a full thickness critical defect using a purified CPCM

Choroid plexus conditioned media was collected as described in example 2, above, purified by dialysis against a 1000 Da MWCO cellulose membrane, and lyophilized as described in group II of figure 5, discussed in example 4, above.

In long-evans rats, a critical defect was created using an 8-mm biopsy punch down to the level of subcutaneous tissue without bandage or autograft. 2 groups containing 10 animals included a treatment (dialyzed conditioned media) and control (lyophilised ENDO-SFM). On each animal, defects were made between the shoulder blades and the excised skin was removed. Animals received a perioperative injection of buprenex and were monitored during recovery. Both groups received topical applications of either dialyzed, lyophilized ENDO-SFM tissue culture medium, or dialyzed lyophilized CP-conditioned medium (DLCM) with dose based on a VEGF loading of approximately 20 ng VEGF. Treatments were applied daily for 10 days, and wounds were explanted on day 14 for histological characterization. Macro photography was used daily to assess and measure wound area. After 4 weeks, animals were euthanized and wound areas were collected for histology.

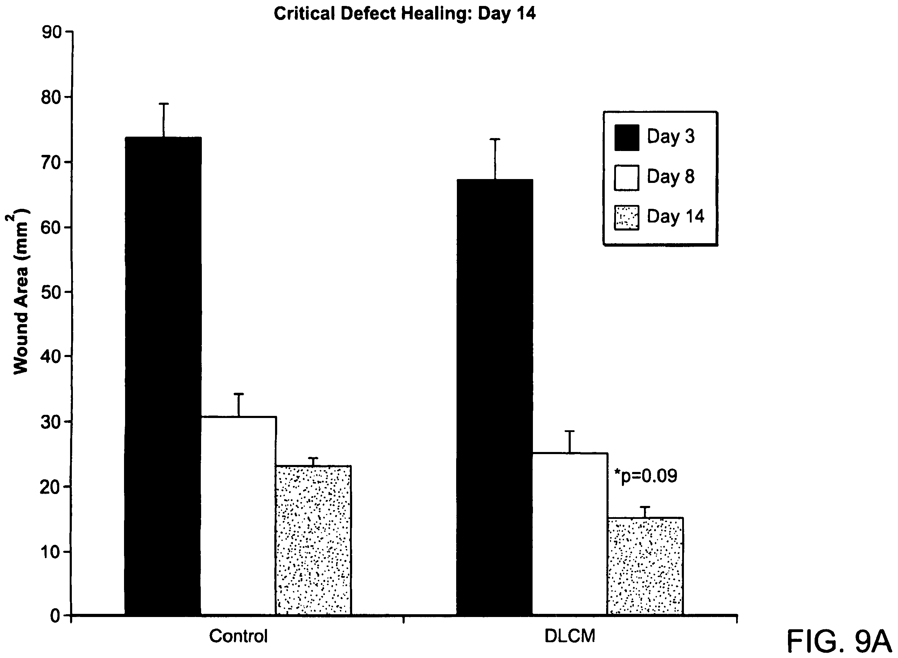

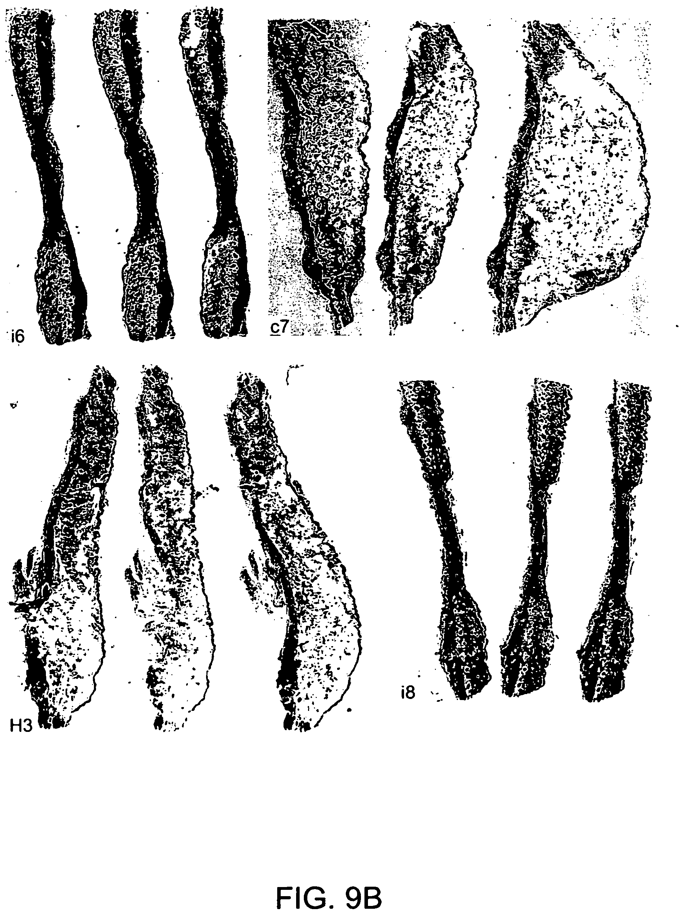

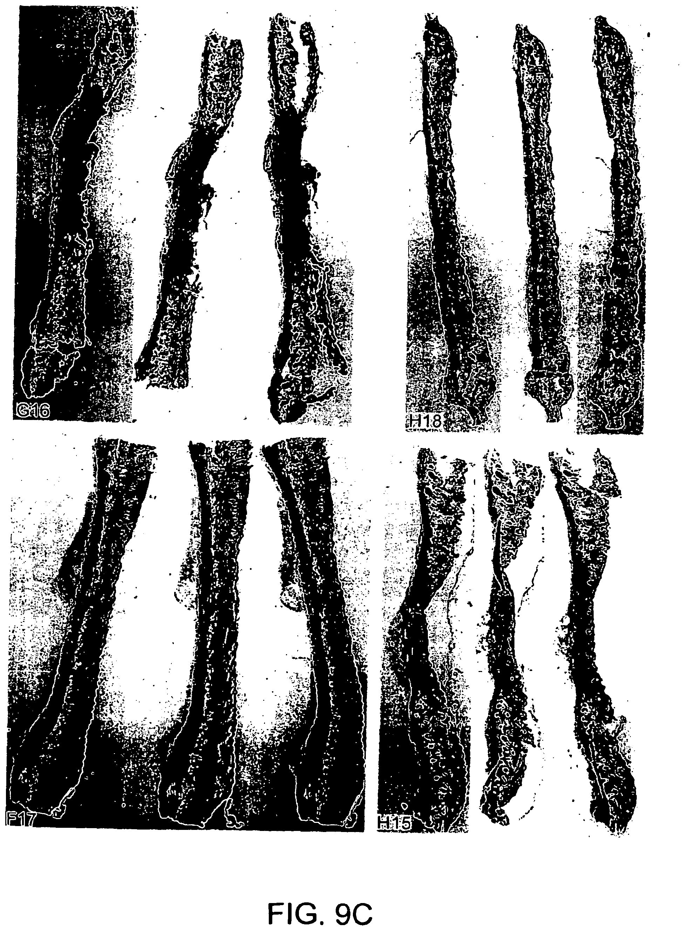

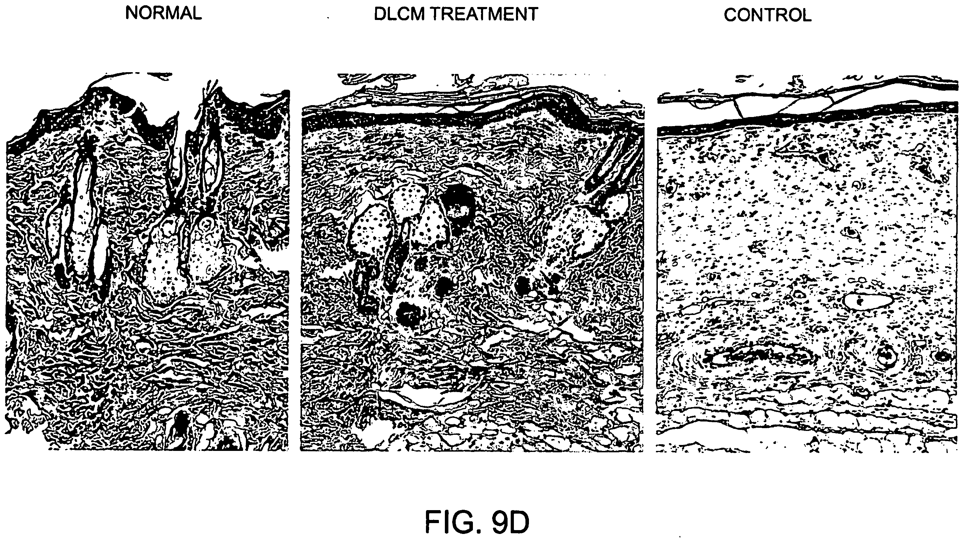

Image analysis of macro photography revealed that both treated and control groups contained wounds that decreased in diameter during wound contraction, although a slight significant improvement was noted in the treatment group (Figure 9A). Gross histologic examination, shown in Figures 9B and 9C, however, reveals a significant increase in subdermal organization in the treated group, including developing hair follicles, vessels, and follicle-associated epithelium. The wounds in the control group (Figure 9B) contain areas that are either inflamed or show severe fibrosis and scarring. At higher magnification (10X) shown in Figure 9D, the full extent of healing can be realized. The left panel shows a section of normal skin adjacent to a control wound, the middle a section of DLCM treated skin, and the right panel shows the control. One obvious aspect of the extent of healing, as noted at the macro level, is the extent of organization and infiltration of advanced remodeling cell types in the middle panel caused by treatment, compared to the far right panel, which contains high levels of fresh collagen and fibroblasts, along with a modicum of neovascularization. The treated group differs mainly from normal tissue in that the hair has not yet emerged through the epidermis, and exists in earlier stages of follicle development. The keratinocyte layer in the extreme top of each section also shows advanced healing, as the control exists with a very shallow, flat layer, compared to the thicker ridges morphology in the treated and normal groups.

### Example 8: Modulation of the cocktail of proteins produced by the choroid plexus, by manipulating culture media composition

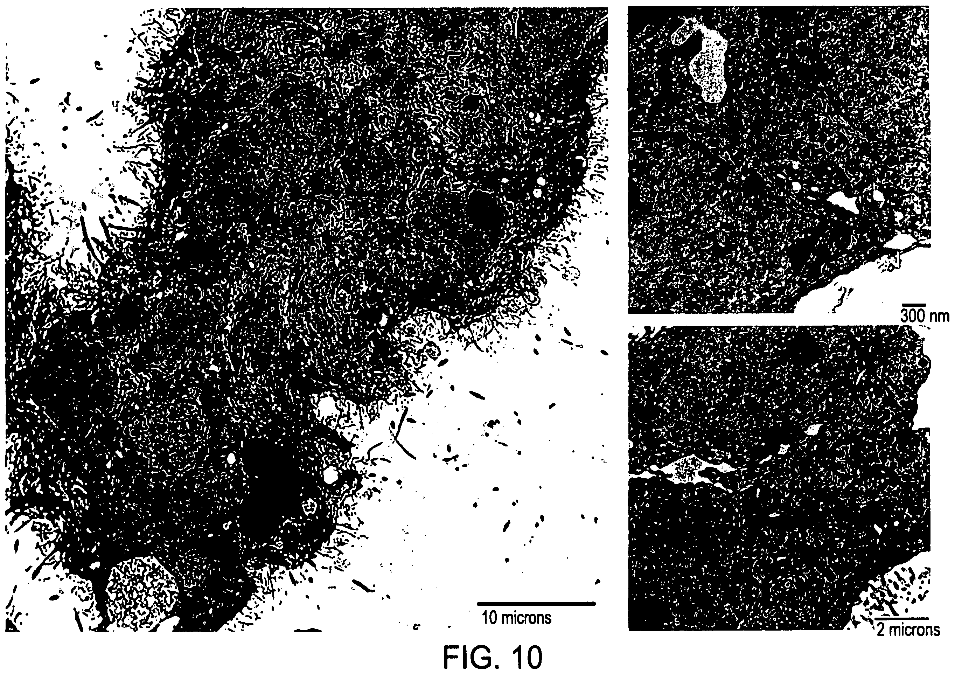

Choroid plexus clusters were isolated and cultured with RPMI-CPN, ENDO-SFM or in RPMI-CPN within a matrix material rich in Collagen IV, laminin and other ECM components. CP clusters normally appear as balls of epithelium with outwardly oriented cilia and attached internally to a matrix composed of connective tissue and other cell types (Figure 10). Cells around the perimeter are rich in tight junctions and show a modicum of secretory vesicles associated with protein release.

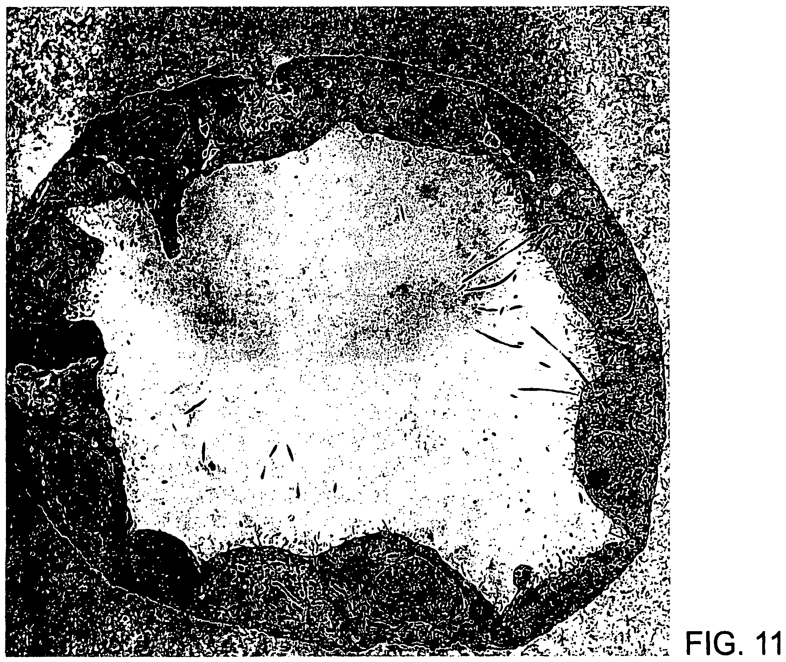

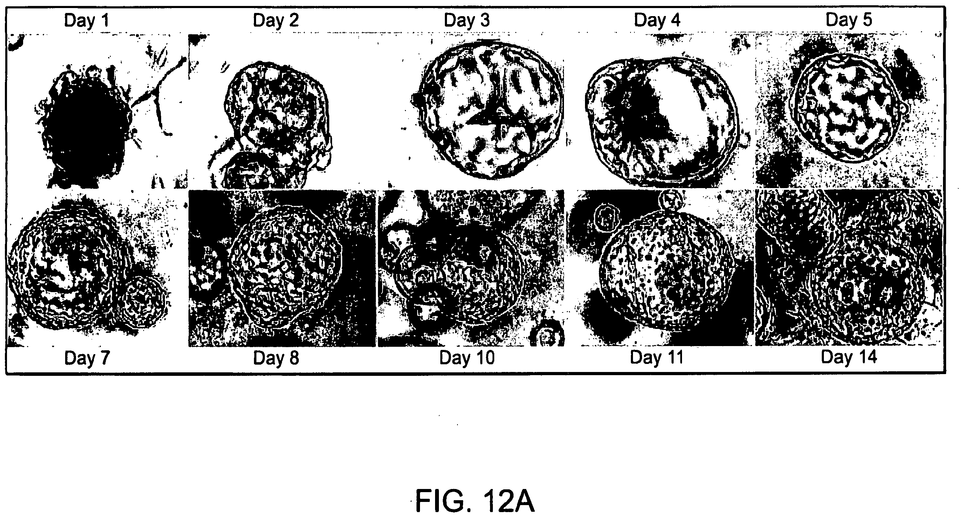

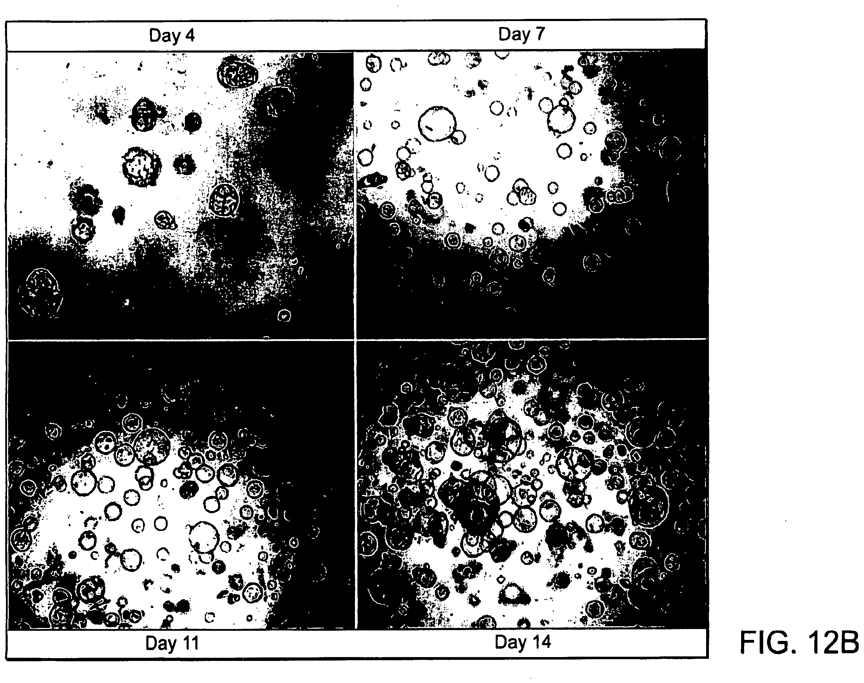

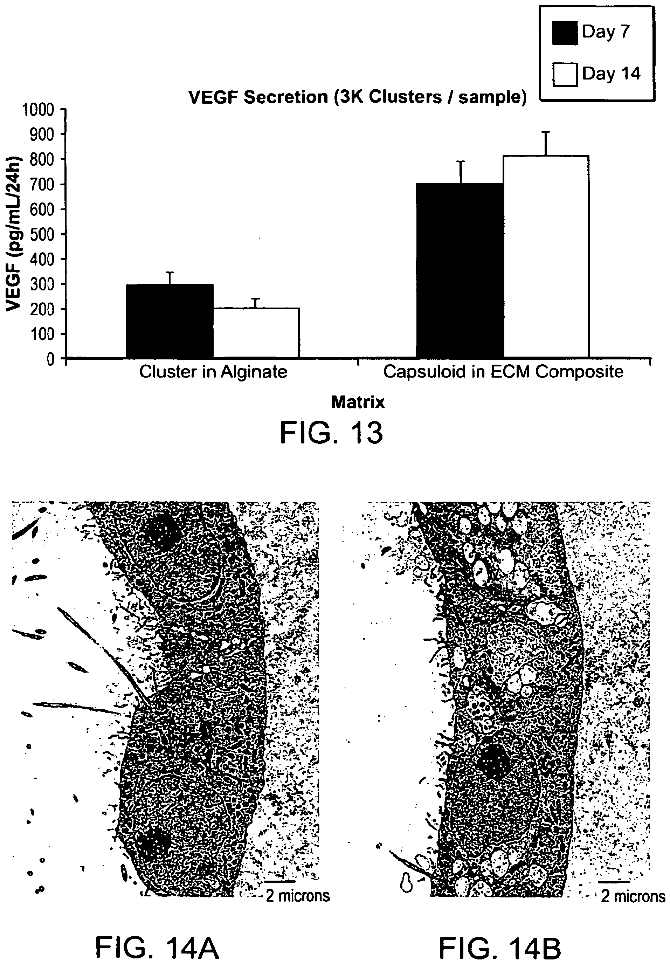

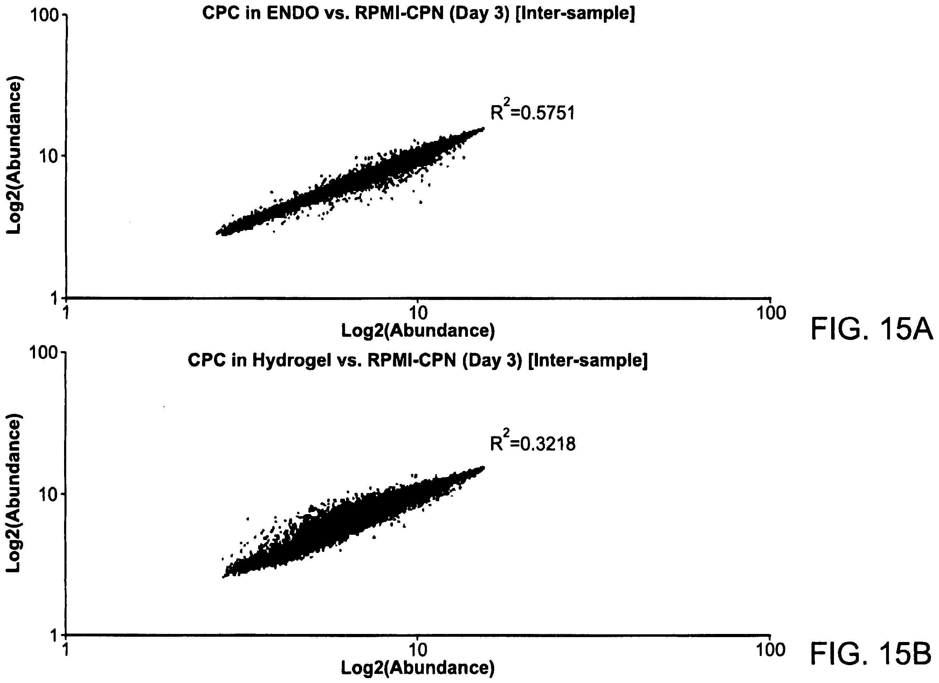

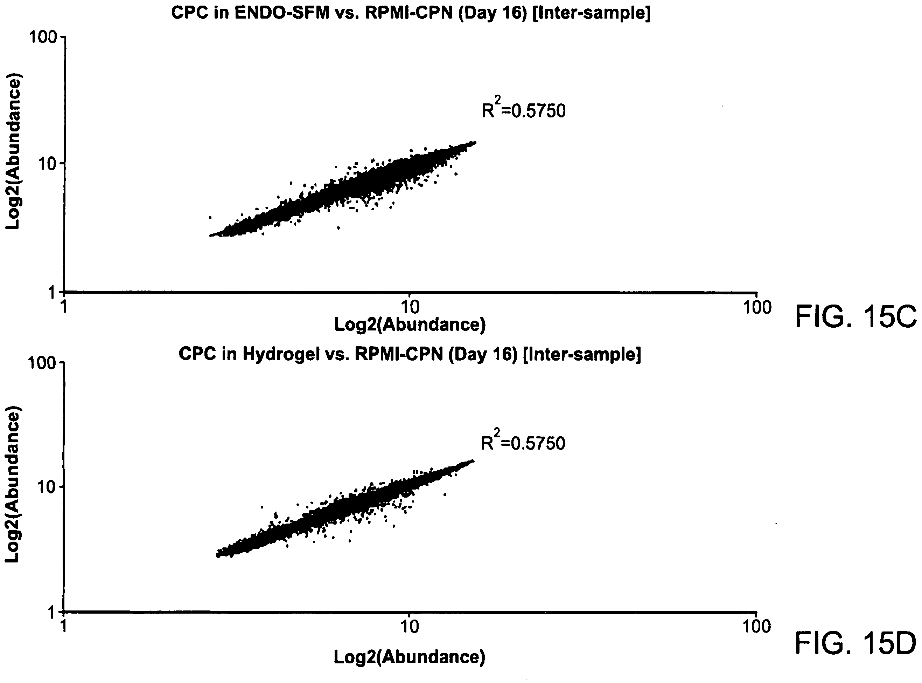

Using an extracellular matrix composition, this morphology was manipulated such that hollow vesicles, or capsuloids, were formed with inward oriented cilia and mirovilli surrounding a fluid-filled internal pocket (Figure 11). These capsuloids varied in size in accordance with the size of the original CP clusters, and enlarged over a 6-10 day timecourse (Figures 12a and 12b). In this process, the epithelium reorganised to form a distinct layer replete with tight junctions, while contaminating cells such as fibroblasts were separated within the matrix. In this configuration, more than twice the level of VEGF was secreted in (Figure 13) and when supplemented with ENDO-SFM, even higher levels of protein vesicle formation was observed (Figure 14b), than in a control capsuloid (Figure 14a). Further, at the level of gene expression, microarray has revealed thousands of changes compared to typical CP culture in RPMI media containing serum. Changes were observed in expression levels related to media composition (Table 2) and capsuloid formation from day 3 (Figure 15a and Figure 15b) to day 16 (Figure 15c and Figure 15d), and as set out in Table 1 below:

**TABLE 1**

| **CAPSULOID GENE CHANGES - RPMI Medium (Changes with respect to CP clusters in RPMI-CPN)** | |
|---|---|
| **Description** | **Fold Change** |
| putative aldo-keto reductase family 1 member C4 | 8.50 |
| GTP binding protein overexpressed in skeletal muscle | 5.93 |
| T-cell receptor alpha chain mRNA C-region, 3' end of cds | 5.55 |
| glutathione S-transferase | 5.02 |
| prepro-beta-defensin 1 | 4.31 |
| carboxypeptidase A1 precursor | 4.22 |
| Antithrombin III | 3.94 |
| neuron-derived orphan receptor-1 alfa | 3.70 |
| Ribosomal protein S 17 | 3.31 |
| v-myc myelocytomatosis viral oncogene homolog (avian) | 3.11 |
| Heat shock protein 70 | 3.05 |
| cytochrome P450 1A1 | 3.02 |
| Palate, lung and nasal epithelium carcinoma associated | 2.95 |
| Serine hydroxymethyltransferase | 2.82 |
| Kruppel-like factor 4 | 2.77 |
| plasminogen activator inhibitor I | 2.75 |
| Polo-like kinase 2 (Drosophila) | 2.71 |
| stefin A1 | 2.57 |
| T-cell receptor alpha chain mRNA C-region, 3' end of cds | 2.48 |
| motilin | 2.47 |
| glutathione S-transferase | 2.46 |
| adrenomedullin | 2.37 |
| Regulator of G-protein signalling 2, 24kDa | 2.30 |
| Sp1 transcription factor | 2.29 |
| growth arrest and DNA-damage-inducible, alpha | 2.27 |
| involucrin | 2.26 |
| Hypothetical protein (5'; clone 2F11) | 2.26 |
| polo-like kinase 3 (Drosophila) | 2.24 |
| lectin-like oxidized LDL receptor-1 | 2.22 |
| 5-hydroxytryptamine (serotonin) receptor 1F | 2.21 |
| activated leukocyte cell adhesion molecule | 2.20 |
| EFNA1 | 2.19 |
| Thymosin beta-4 | 2.19 |
| paired box gene 3 (Waardenburg syndrome 1) | 2.18 |
| Hypothetical protein (5'; clone 7H1) | 2.18 |
| Orosomucoid 1 | 2.17 |
| metallothionein-III | 2.16 |
| olfactory receptor | 2.14 |
| Hoxa10-like mRNA, partial sequence /// Homeobox protein A10 | 2.14 |
| psoriasis susceptibility 1 candidate 2 | 2.13 |
| Estrogen sulfotransferase | 2.13 |
| T-cell receptor alpha chain mRNA C-region, 3' end of cds | 2.13 |
| T, brachyury homolog (mouse) | 2.12 |
| collagen, type IV, alpha 3 (Goodpasture antigen) | 2.12 |
| olfactory receptor | 2.11 |
| T-cell receptor gamma and delta constant region | 2.11 |
| elastase 2, neutrophil | 2.11 |
| CMP-N-acetylneuraminate monooxygenase | 2.11 |
| Hypothetical protein (5'; clone 7A4) | 2.11 |
| sodium channel, voltage-gated, type II, alpha subunit | 2.11 |
| CD28 antigen | 2.09 |
| Rho family GTPase 3 | 2.09 |
| Glycerine aldehyde 3-phosphate dehydrogenase (GAPDH) | 2.08 |
| cytochrome P450 2C36 | 2.07 |
| oxytocin receptor | 2.07 |
| glutathione S-transferase | 2.07 |
| Fatty acid binding protein 5 | 2.07 |
| angiopoietin-like 4 | 2.07 |
| Tumor-associated calcium signal transducer 1 | 2.06 |
| T-cell receptor alpha chain mRNA C-region, 3' end of cds | 2.06 |
| thioredoxin | 2.04 |
| Complement C1qC | 2.03 |
| matrix metallopeptidase 7 (matrilysin, uterine) | 2.03 |
| calcitonin receptor-stimulating peptide-3 | 2.03 |
| Chemokine | 2.02 |
| T-cell receptor alpha chain mRNA C-region, 3' end of cds | 2.01 |
| T-cell receptor alpha chain mRNA C-region, 3' end of cds | 2.01 |
| ribosomal protein, large, P1 | 2.00 |
| Brain multidrug resistance protein | 2.00 |
| SH3 mRNA, 3' UTR | 2.00 |
| sodium/hydrogen exchanger isoform 3 | 1.99 |
| T-cell receptor alpha chain mRNA C-region, 3' end of cds | 1.99 |
| T-cell receptor alpha chain mRNA C-region, 3' end of cds /// TCR-a mRNA for T ce | 1.99 |
| short type I interferon | 1.99 |
| arachidonate 15-lipoxygenase | 1.98 |
| acidic fibroblast growth factor | 1.98 |
| Sp1 transcription factor | 1.98 |
| Cytochrome P450 19A3 | 1.98 |
| Unc-5 homolog C (C. elegans) | 1.98 |
| deiodinase, iodothyronine, type II | 1.97 |
| sarcoendoplasmic reticulum calcium ATPase | 1.97 |
| Annexin A1 | 1.97 |
| T-cell receptor alpha chain mRNA C-region, 3' end of cds | 1.97 |
| somatostatin receptor subtype 3 | 1.97 |
| Chromosome 17 clone pkmCon86, mRNA sequence | 1.97 |
| Microsomal glutathione S-transferase 1 | 1.97 |
| CD28 antigen | 1.96 |
| pancreatic polypeptide receptor 1 | 1.96 |
| bone morphogenetic protein 15 | 1.96 |
| tissue inhibitor of metalloproteinase-4 | 1.96 |
| DEAD (Asp-Glu-Ala-Asp) box polypeptide 4 | 1.96 |
| Pregnancy-associated glycoprotein 6 | 1.96 |
| amphiregulin (schwannoma-derived growth factor) | 1.96 |
| interleukin 1, beta | 1.96 |
| integrin, alpha M | 1.95 |
| Tyrosinase-related protein 1 | 1.95 |
| hairless | 1.94 |
| titin | 1.94 |
| CD80 molecule | 1.94 |
| chymosin | 1.92 |
| protein kinase C, beta 1 | 1.92 |
| proteasome (prosome, macropain) subunit, beta type, 10 | 1.92 |
| Tropomodulin 3 (ubiquitous) | 1.92 |
| Matrix metallopeptidase 1 (interstitial collagenase) | 1.92 |
| olfactory receptor | 1.92 |
| heme oxygenase | 1.91 |
| fucosyltransferase 1 (galactoside 2-alpha-L-fucosyltransferase, H blood group) | 1.91 |
| Tropomyosin 3 | 1.91 |
| Leydig insulin-like hormone | 1.91 |
| Creatine kinase, muscle | 1.90 |
| peptidoglycan recognition protein S isoform | 1.90 |
| T-cell receptor alpha chain mRNA C-region, 3' end of cds | 1.90 |
| uroplakin 3 | 1.89 |
| Hypothetical protein (5'; clone 2C4) | 1.89 |
| POU | 1.89 |
| ribosomal protein, large, P1 | 1.89 |
| Rearranged T-cell receptor delta-chain/Vdelta5.1-Ddeltas-Jdelta1 | 1.89 |
| TCR-a mRNA for T cell receptor alpha chain, clone:PTA014 | 1.89 |
| paraoxonase 3 | 1.88 |
| fatty acid binding protein 5 | 1.88 |
| phosphodiesterase 4A, cAMP-specific (phosphodiesterase E2 dunce homolog, Drosoph | 1.88 |
| malic enzyme 1, NADP(+)-dependent, cytosolic | 1.88 |
| calcitonin receptor-stimulating peptide-2 | 1.88 |
| Fc fragment of IgG, low affinity IIIb, receptor (CD16b) | 1.88 |
| interleukin-17 | 1.88 |
| Interleukin 1, beta | 1.87 |
| FETUIN protein | 1.87 |
| Immunoglobulin VDJ region | 1.87 |
| CD 14 antigen | 1.87 |
| T-cell receptor alpha chain mRNA C-region, 3' end of cds | 1.87 |
| CD69 molecule | 1.87 |
| transcription factor Sp1 | 1.87 |
| Antithrombin III | 1.86 |
| UPF3 regulator of nonsense transcripts-like protein B | 1.86 |
| Palate, lung and nasal epithelium carcinoma associated | 1.86 |
| myoglobin | 1.85 |
| low density lipoprotein receptor | 1.85 |
| chemokine (C-C motif) receptor 3 | 1.85 |
| Transthyretin | 1.84 |
| T-cell receptor alpha chain mRNA C-region, 3' end of cds | 1.84 |
| pregnancy-associated plasma protein-A | 1.84 |
| neuropeptide Y receptor Y2 | 1.84 |
| killer cell lectin-like receptor subfamily A, member 1 | 1.84 |
| sulfotransferase family, cytosolic, 2A, dehydroepiandrosterone (DHEA)-preferring | 1.84 |
| T-cell receptor beta chain mRNA C-region, 3' end of cds | 1.84 |
| RNA helicase | 1.84 |
| transforming growth factor, beta 3 | 1.84 |
| CD3d molecule, delta (CD3-TCR complex) | 1.83 |
| Soluble angiotensin-binding protein (sABP), SINE senquence | 1.83 |
| insulin-like growth factor binding protein 1 | 1.83 |
| gonadotropin-releasing hormone receptor | 1.83 |
| T-cell receptor gamma and delta constant region | 1.83 |
| Hypothetical protein (5'; clone 7A4) | 1.83 |
| calsequestrin | 1.82 |
| olfactory receptor | 1.82 |
| Cytochrome P450 19A1 | 1.82 |
| olfactory receptor | 1.82 |
| cholinergic receptor, muscarinic 1 | 1.82 |
| T-cell receptor alpha chain mRNA C-region, 3' end of cds | 1.82 |
| Prostaglandin-endoperoxide synthase 1 | 1.81 |
| T-cell receptor alpha chain mRNA C-region, 3' end of cds | 1.81 |
| ficolin | 1.81 |
| myxovirus (influenza virus) resistance 2 (mouse) | 1.81 |
| Ribosomal protein L12 | 1.81 |
| chemokine (C-X-C motif) receptor 6 | 1.81 |
| Serum amyloid A2 | 1.81 |
| somatostatin receptor subtype 4 | 1.80 |
| Mox2 protein | 1.80 |
| GATA binding protein 4 | 1.80 |
| Isolate K8253 TCR-delta chain CDR3 region (TCRD) | 1.80 |
| carbonyl reductase 1 | 1.80 |
| interferon-gamma | 1.80 |

| **DOWNREGULATED GENES** | |
|---|---|
| **Description** | **Fold Change** |
| Amylase, alpha 2B (pancreatic) | -13.75 |
| Eukaryotic translation elongation factor 1 gamma | -11.59 |
| Complement ClqC | -10.16 |
| Tropomodulin 3 (ubiquitous) | -9.86 |
| solute carrier organic anion transporter family, member 1A2 | -8.75 |
| protein phosphatase 1 catalytic subunit gamma isoform | -7.94 |
| Acyl-CoA oxidase | -7.36 |
| Nuclear factor of activated T-cells, cytoplasmic, calcineurin-dependent 1 | -7.27 |
| complement component 1, q subcomponent, B chain | -7.17 |
| interleukin 13 receptor, alpha 1 | -7.13 |
| Caveolin 1 | -6.14 |
| Putative membrane steroid receptor | -6.11 |
| Optineurin /// Tumor-associated calcium signal transducer 1 | -6.08 |
| Putative aldo-keto reductase family 1 member C4 | -5.88 |
| Arginyl-tRNA synthetase | -5.88 |
| Hypoxia-inducible factor 1, alpha subunit (basic helix-loop-helix transcription | -5.86 |
| protein phosphatase 1, catalytic subunit, beta isoform | -5.76 |
| Ribosomal protein L10 | -5.47 |
| Optineurin | -5.45 |
| coxsackie-adenovirus-receptor homolog | -5.13 |
| caspase 3, apoptosis-related cysteine peptidase | -4.72 |
| Histone H1.3-like protein | -4.67 |
| Finkel-Biskis-Reilly murine sarcoma virus (FBR-MuSV) ubiquitously | -4.62 |
| expressed (fox Sterol-C4-methyl oxidase-like | -4.61 |
| 5-hydroxytryptamine (serotonin) receptor 2C | -4.61 |
| radixin | -4.49 |
| Insulin induced gene 1 | -4.44 |
| zinc finger protein 313 | -4.37 |
| connective tissue growth factor | -4.34 |
| Protein phosphatase 1, regulatory (inhibitor) subunit 12A | -4.33 |
| RAB14, member RAS oncogene family | -4.31 |
| Calpain, small subunit 1 | -4.14 |
| solute carrier organic anion transporter family, member 1A2 | -4.10 |
| FGF receptor 2IIIc | -3.88 |
| Palate, lung and nasal epithelium carcinoma associated | -3.87 |
| Eukaryotic translation elongation factor 1 alpha 1 | -3.83 |
| Eukaryotic translation elongation factor 1 gamma | -3.79 |
| Collagen, type III, alpha 1 (Ehlers-Danlos syndrome type IV, autosomal dominant) | -3.78 |
| Calcitonin receptor-like | -3.66 |
| glutamate-ammonia ligase (glutamine synthetase) | -3.58 |
| zinc finger protein 265 | -3.58 |
| Destrin | -3.52 |
| lipoprotein lipase | -3.51 |
| Palate, lung and nasal epithelium carcinoma associated | -3.50 |
| Insulin induced gene 1 | -3.48 |
| protein phosphatase 1, catalytic subunit, beta isoform | -3.47 |
| guanylate cyclase 1, soluble, beta 3 | -3.45 |
| Transmembrane protein 59 | -3.42 |
| caveolin 1 | -3.42 |
| Eukaryotic translation elongation factor 1 gamma | -3.38 |
| Neuropeptide Y receptor Y1 | -3.37 |
| Putative aldo-keto reductase family 1 member C4 | -3.32 |
| glutaminase | -3.28 |
| UDP-GIcNAc:betaGal beta-1,3-N-acetylglucosaminyltransferase 5 | -3.26 |
| cyclin-dependent kinase inhibitor 1B (p27, Kip1) | -3.24 |
| lysozyme | -3.23 |
| AMP-activated protein kinase alpha 2 | -3.22 |
| succinate dehydrogenase complex, subunit A, flavoprotein (Fp) | -3.20 |
| Thioredoxin | -3.19 |
| splicing factor, arginine/serine-rich 11 | -3.19 |
| Tubulin alpha | -3.19 |
| Actin-related protein 3 | -3.17 |
| alveolar macrophage-derived chemotactic factor-I | -3.14 |
| Coxsackie-adenovirus-receptor homolog | -3.08 |
| actin, beta | -3.07 |
| Cofilin 2 | -3.05 |
| Tyrosine 3-monooxygenase/tryptophan 5-monooxygenase activation protein, zeta pol | -3.05 |
| Tropomodulin 3 (ubiquitous) | -3.04 |
| Palate, lung and nasal epithelium carcinoma associated | -2.99 |
| actin, beta | -2.99 |
| son3 protein | -2.98 |
| double stranded RNA-dependent protein kinase | -2.92 |
| Insulin-induced membrane protein 2 (INSIG2) | -2.92 |
| Protein phosphatase 1, regulatory (inhibitor) subunit 12A | -2.91 |
| Coxsackie-adenovirus-receptor homolog | -2.91 |
| extracellular related kinase 3 | -2.84 |
| Solute carrier family 35 (UDP-N-acetylglucosamine (UDP-GlcNAc) transporter), mem | -2.83 |
| exportin 1 (CRM1 homolog, yeast) | -2.82 |
| calcium/calmodulin-dependent protein kinase (CaM kinase) II delta | -2.78 |
| Coxsackie-adenovirus-receptor homolog | -2.77 |
| Scavenger receptor class B member 2 | -2.77 |
| O-linked N-acetylglucosamine (GlcNAc) transferase (UDP-N-acetylglucosamine:polyp | -2.76 |
| tropomyosin 1 (alpha) | -2.75 |
| Endothelial PAS domain protein 1 | -2.75 |
| Inhibin, beta A (activin A, activin AB alpha polypeptide) | -2.71 |
| aquaporin 1 | -2.71 |
| Tropomodulin 3 (ubiquitous) | -2.70 |
| vascular cell adhesion molecule | -2.69 |
| similar to tigger transposable element derived 4 | -2.68 |
| amylase, alpha 2B (pancreatic) | -2.68 |
| protein phosphatase 2 (formerly 2A), catalytic subunit, alpha isoform | -2.67 |
| polypeptide chain elongation factor 1 alpha | -2.67 |
| transforming growth factor, beta 2 | -2.66 |
| Hypothetical protein LOC733658 | -2.66 |
| Calcineurin A protein | -2.65 |
| 3-hydroxy-3-methylglutaryl coenzyme A reductase/HMG-CoA reductase | -2.64 |
| Splicing factor, arginine/serine-rich 6 | -2.63 |
| complement component 1, q subcomponent, A chain | -2.61 |
| Cell division cycle 42 | -2.61 |
| secreted phosphoprotein 1 (osteopontin, bone sialoprotein I, early T-lymphocyte | -2.61 |
| polypyrimidine tract-binding protein | -2.60 |
| Vitamin D3 25-Hydroxylase | -2.59 |
| Somatostatin | -2.59 |
| protein kinase, cAMP-dependent, regulatory, type II, alpha | -2.59 |
| CD59 molecule, complement regulatory protein | -2.59 |
| pleiotrophic factor beta | -2.59 |
| PRA1 family protein-like protein | -2.58 |
| Tubulin alpha | -2.58 |
| Tropomodulin 3 (ubiquitous) | -2.57 |
| High-affinity copper uptake protein | -2.56 |
| solute carrier family 7 (cationic amino acid transporter, y+ system), | -2.51 |
| member 1 | |
| Rho family GTPase 3 | -2.51 |
| RAP2A | -2.50 |
| Splicing factor, arginine/serine-rich 6 | -2.50 |
| keratinocyte growth factor receptor | -2.49 |
| O-linked N-acetylglucosamine (GlcNAc) transferase (UDP-N-acetylglucosamine:polyp | -2.49 |
| Calumenin | -2.48 |
| microphthalmia-associated transcription factor | -2.48 |
| mitogen-activated protein kinase 9 | -2.47 |
| Melanoma cell adhesion molecule | -2.47 |
| Eukaryotic translation elongation factor 1 alpha 1 | -2.47 |
| rab geranylgeranyltransferase, beta subunit | -2.46 |
| Transmembrane protein 59 | -2.44 |
| Tumor-associated calcium signal transducer 1 | -2.44 |
| high-affinity copper uptake protein | -2.44 |
| Quaking homolog, KH domain RNA binding (mouse) | -2.42 |
| Calpain, small subunit 1 | -2.42 |
| actin-related protein 3 | -2.40 |
| Nuclear receptor coactivator 1 | -2.39 |
| ID4 | -2.39 |
| caveolin 1 | -2.39 |
| Protein phosphatase 1, regulatory (inhibitor) subunit 12A | -2.39 |
| ATP synthase, H+ transporting, mitochondrial F0 complex, subunit G | -2.38 |
| connexin 43 | -2.37 |
| interferon-related developmental regulator 1 | -2.37 |
| PRA1 family protein-like protein | -2.36 |
| Ribosomal protein L23 | -2.33 |
| Tropomodulin 3 (ubiquitous) | -2.33 |
| Calpain, small subunit 1 | -2.33 |
| Radixin | -2.32 |
| glutamine-fructose-6-phosphate transaminase 1 | -2.32 |
| protein phosphatase 1 catalytic subunit alpha isoform | -2.31 |
| Collagen, type I, alpha 1 | -2.31 |
| Disintegrin-metalloproteinase precursor | -2.31 |
| Immunoglobulin alpha heavy chain constant region (IgA C alpha) | -2.31 |
| Collagen, type III, alpha 1 (Ehlers-Danlos syndrome type IV, autosomal dominant) | -2.31 |
| Tropomodulin 3 (ubiquitous) | -2.31 |
| pinin, desmosome associated protein | -2.31 |
| Gi-alpha-3 protein | -2.30 |
| inositol(myo)-1(or 4)-monophosphatase 1 | -2.30 |
| Eukaryotic translation elongation factor 1 gamma | -3.29 |
| Cytochrome P450 21-hydroxylase | -2.29 |
| Tubulin alpha | -2.28 |
| Caveolin 1 | -2.28 |
| amylase, alpha 2B (pancreatic) | -2.28 |
| FBXO 32 | -2.28 |
| Palate, lung and nasal epithelium carcinoma associated | -2.27 |
| Karyopherin alpha 3 | -2.26 |
| Palate, lung and nasal epithelium carcinoma associated | -2.26 |
| Tubulin alpha | -2.26 |
| Succinyl-CoA:alpha-ketoacid coenzyme A transferase | -2.25 |
| O-linked N-acetylglucosamine (GlcNAc) transferase (UDP-N-acetylglucosamine:polyp | -2.24 |
| Finkel-Biskis-Reilly murine sarcoma virus (FBR-MuSV) ubiquitously | -2.23 |
| expressed (fox | |
| Membrane cofactor protein | -2.22 |
| Splicing factor, arginine/serine-rich 11 | -2.22 |
| peroxisome proliferator activated receptor,gamma,coactivator 1 | -2.22 |
| Tropomodulin 3 (ubiquitous) | -2.22 |
| Lipoprotein lipase | -2.21 |
| mothers against decapentaplegic homolog 1 | -2.21 |
| protein kinase, cAMP-dependent, catalytic, beta | -2.21 |
| pinin, desmosome associated protein | -2.21 |
| Ras-related protein Rab-11A | -2.20 |
| Chromosome 17 clone pkmCon73, mRNA sequence | -2.18 |
| solute carrier family 22 (organic anion transporter), member 8 | -2.18 |
| Low density lipoprotein receptor | -2.17 |
| proteasome (prosome, macropain) activator subunit 3 (PA28 gamma; Ki) | -2.17 |
| Cytochrome P450 17A1 | -2.16 |
| Transcription factor A | -2.16 |
| UPF3 regulator of nonsense transcripts-like protein B | -2.15 |
| claudin-1 protein | -2.15 |
| Palate, lung and nasal epithelium carcinoma associated | -2.15 |
| cAMP-regulated phosphoprotein | -2.15 |
| Inhibin, beta A (activin A, activin AB alpha polypeptide) | -2.15 |
| splicing factor, arginine/serine-rich 11 | -2.15 |
| HPCA | -2.15 |
| Tubulin alpha | -2.14 |
| Putative membrane steroid receptor | -2.14 |
| insulin-like growth factor 2 (somatomedin A) | -2.13 |
| alpha-1,3-galactosyltransferase | -2.13 |
| sterol-C4-methyl oxidase-like | -2.12 |
| O-linked N-acetylglucosamine (GlcNAc) transferase (UDP-N-acetylglucosamine:polyp | -2.11 |
| splicing factor, arginine/serine-rich 11 | -2.11 |
| Extracellular related kinase 3 | -2.10 |
| 90-kDa heat shock protein | -2.10 |
| Protein S | -2.09 |
| TrkB protein | -2.09 |
| Fatty acid synthase | -2.09 |
| acyl-CoA synthetase long-chain family member 4 | -2.08 |
| Nuclear factor of activated T-cells, cytoplasmic, calcineurin-dependent 1 | -2.07 |
| guanine nucleotide binding protein (G protein), alpha inhibiting activity polype | -2.07 |
| hypothetical protein LOC733652 | -2.06 |
| ribose 5-phosphate isomerase-like protein | -2.06 |
| Integral membrane protein | -2.06 |
| Claudin-2 | -2.06 |
| RAP2A | -2.05 |
| myosin VI | -2.05 |
| Tropomodulin 3 (ubiquitous) | -2.04 |
| fibroblast growth factor 2 | -2.04 |
| ROD1 regulator of differentiation 1 | -2.03 |
| Finkel-Biskis-Reilly murine sarcoma virus (FBR-MuSV) ubiquitously | -2.03 |
| expressed (fox | |
| Calpain, small subunit 1 | -2.03 |
| Calmodulin | -2.03 |
| tropomodulin 3 (ubiquitous) | -2.03 |
| Superoxide dismutase 2, mitochondrial | -2.03 |
| Polymerase (RNA) II (DNA directed) polypeptide B, 140kDa | -2.02 |
| Occludin | -2.01 |
| Glutathione S-transferase | -2.01 |
| Optineurin | -2.01 |
| RAB1B, member RAS oncogene family | -2.01 |
| vascular endothelial growth factor | -2.01 |
| Tropomodulin 3 (ubiquitous) | -2.01 |
| Activated leukocyte cell adhesion molecule | -2.00 |
| Eukaryotic translation elongation factor 1 gamma | -2.00 |
| complement component 3 | -1.98 |
| splicing factor, arginine/serine-rich 1 (splicing factor 2, alternate splicing f | -1.98 |
| SLA-2 mRNA for MHC class I antigen, partial cds, allele: | -1.97 |
| SLA-2*03 | |
| Optineurin | -1.97 |
| Tropomodulin 3 (ubiquitous) | -1.97 |
| Eukaryotic translation elongation factor 1 alpha 1 | -1.97 |
| Splicing factor, arginine/serine-rich 6 | -1.97 |
| acyl-CoA synthetase long-chain family member 4 | -1.96 |
| Eukaryotic translation elongation factor 1 gamma | -1.96 |
| Unidentified hepatic protein mRNA | -1.95 |
| Eukaryotic translation elongation factor 1 gamma | -1.95 |
| Albumin | -1.95 |
| Transcription factor A | -1.95 |
| Optineurin | -1.94 |
| insulin-like growth factor 2 (somatomedin A) | -1.93 |
| Palate, lung and nasal epithelium carcinoma associated | -1.93 |
| alpha-1,3-galactosyltransferase | -1.93 |
| Glutathione S-transferase | -1.93 |
| Hn-RNA, clone b3155 | -1.92 |
| Calponin 2 | -1.92 |
| Collagen, type III, alpha 1 (Ehlers-Danlos syndrome type IV, autosomal dominant) | -1.91 |
| Tropomodulin 3 (ubiquitous) | -1.91 |
| PNAS-4 protein | -1.91 |
| Sterol regulatory element binding protein 2 | -1.91 |
| Tubulin alpha | -1.91 |
| Karyopherin alpha 2 | -1.91 |
| ID4 | -1.91 |
| Epoxide hydrolase | -1.91 |
| HLA-B associated transcript 1 | -1.90 |
| Optineurin | -1.90 |
| RAB1B, member RAS oncogene family | -1.90 |
| Ribosomal protein L10 | -1.90 |
| Optineurin | -1.89 |
| TNF receptor-associated factor 6 | -1.89 |
| stearoyl-CoA desaturase | -1.89 |
| Protein kinase, cAMP-dependent, catalytic, beta | -1.89 |
| Eukaryotic translation elongation factor 1 gamma | -1.88 |
| sarcoplasmic/endoplasmic-reticulum Ca(2+) pump gene 2 | -1.88 |
| fatty acid binding protein 4, adipocyte | -1.88 |
| adenylate kinase 5 | -1.87 |
| Tubulin alpha | -1.87 |
| COX7C1 (COX7C1) | -1.87 |
| ATPase, Ca++ transporting, plasma membrane 1 | -1.87 |
| Glycerol-3-phosphate dehydrogenase | -1.87 |
| putative inhibitor of apoptosis | -1.86 |
| Ubiquitin-activating enzyme E1 | -1.86 |
| RAN, member RAS oncogene family | -1.86 |
| Eukaryotic translation elongation factor 1 alpha 1 | -1.86 |
| Inhibin, beta A (activin A, activin AB alpha polypeptide) | -1.85 |
| Rho family GTPase 3 | -1.85 |
| Voltage-dependent anion channel 2 | -1.85 |
| ATPase, Na+/K+ transporting, beta 1 polypeptide | -1.85 |
| Splicing factor, arginine/serine-rich 11 | -1.85 |
| fatty acid desaturase 2 | -1.84 |
| Splicing factor, arginine/serine-rich 10 (transformer 2 homolog, Drosophila) | -1.84 |
| secretory carrier membrane protein 1 | -1.84 |
| Optineurin | -1.84 |
| insulin-like growth factor 2 (somatomedin A) | -1.84 |
| Abhydrolase domain containing 5 | -1.84 |
| Complement component 1, r subcomponent | -1.84 |
| insulin-like growth factor 2 (somatomedin A) | -1.84 |
| Heparin binding protein | -1.83 |
| Splicing factor, arginine/serine-rich 10 (transformer 2 homolog, Drosophila) | -1.83 |
| Proliferating cell nuclear antigen | -1.83 |
| fibrillin 1 | -1.83 |
| Glutathione S-transferase | -1.83 |
| Tumor-associated calcium signal transducer 1 | -1.83 |
| Transthyretin | -1.83 |
| SAR1a gene homolog 2 | -1.82 |
| calpastatin | -1.82 |
| voltage-dependent anion channel 1 | -1.82 |
| transcription factor TZP | -1.81 |
| son3 protein | -1.81 |
| Chromosome 17 clone pkmCon24, mRNA sequence | -1.81 |
| Tubulin alpha | -1.81 |
| Phosphoglucomutase 1 | -1.80 |
| transcription factor TZP | -1.80 |
| integrin beta-1 subunit | -1.80 |
| Glutathione S-transferase | -1.80 |
| Calpain, small subunit 1 | -1.80 |
| tissue factor | -1.80 |

**TABLE 2**

| **GENE CHANGES - ENDO-SFM Containing Clusters (Changes with respect to clusters in RPMI-CPN)** | |
|---|---|
| **Description** | **Fold Change** |
| Haptocorrin | 43.16 |
| fatty acid binding protein 4, adipocyte | 23.21 |
| ATP1A2 mRNA, 3'UTR, allele 1 | 12.90 |
| Tropomodulin 3 (ubiquitous) | 8.37 |
| galectin-1 | 7.35 |
| Tyrosinase-related protein 1 | 6.37 |
| lipoprotein lipase | 5.99 |
| matrix Gla protein | 5.30 |
| Tropomodulin 3 (ubiquitous) | 5.25 |
| Collagen, type III, alpha 1 (Ehlers-Danlos syndrome type IV, autosomal dominant) | 5.22 |
| Lipoprotein lipase | 5.18 |
| Group VII phospholipase A2 | 5.11 |
| Tropomodulin 3 (ubiquitous) | 4.92 |
| haptocorrin | 4.82 |
| Tropomodulin 3 (ubiquitous) | 4.79 |
| pigment epithelium-derived factor | 4.55 |
| Tubulin alpha | 4.50 |
| caveolin 1 | 4.26 |
| Beta-2-microglobulin | 4.20 |
| caveolin 1 | 4.15 |
| complement component 5 | 4.11 |
| Chemokine | 3.95 |
| Na,K-ATPase alpha 2 subunit | 3.86 |
| Fatty acid binding protein 5 | 3.80 |
| Tubulin alpha | 3.67 |
| fatty acid binding protein 5 | 3.66 |
| alpha-1,3-galactosyltransferase | 3.55 |
| Chemokine | 3.54 |
| TrkB protein | 3.49 |
| fibroblast growth factor 2 | 3.47 |
| collagen, type III, alpha 1 (Ehlers-Danlos syndrome type IV, autosomal dominant) | 3.47 |
| alpha-actinin-2-associated LIM protein | 3.41 |
| odd homeobox 1 protein | 3.41 |
| chemokine (C-C motif) receptor 1 | 3.31 |
| Tropomyosin 3 | 3.29 |
| caveolin 1 | 3.28 |
| alpha-1,3-galactosyltransferase. | 3.21 |
| Collagen, type I, alpha 1 | 3.16 |
| Fatty acid binding protein 5 | 3.14 |
| Chromosome 17 clone pkmCon46, mRNA sequence | 3.05 |
| Thioredoxin interacting protein | 3.01 |
| alpha-1,3-galactosyltransferase | 2.98 |
| Somatostatin | 2.94 |
| Lipoprotein lipase | 2.90 |
| Lipoprotein lipase | 2.90 |
| cathepsin K | 2.83 |
| Fibronectin | 2.80 |
| UDP-Gal:beta-GIcNAc beta-1,3-galactosyltransferase 3 | 2.69 |
| connexin 43 | 2.55 |
| Membrane-associated protein 17 | 2.39 |
| metallothionein-III | 2.38 |
| complement component 1, s subcomponent | 2.35 |
| Occludin | 2.29 |
| putative aldo-keto reductase family 1 member C4 | 2.29 |
| Inhibin, beta A (activin A, activin AB alpha polypeptide) | 2.28 |
| glutathione S-transferase | 2.28 |
| chemokine (C-X-C motif) ligand 2 | 2.25 |
| transforming growth factor, beta 2 | 2.25 |
| Calpain, small subunit 1 | 2.23 |
| Collagen, type III, alpha 1 (Ehlers-Danlos syndrome type IV, autosomal dominant) | 2.22 |
| fatty acid binding protein 3, muscle and heart (mammary-derived growth inhibitor | 2.21 |
| acyl-CoA synthetase long-chain family member 4 | 2.20 |
| pro-melanin-concentrating hormone | 2.19 |
| CD163 molecule | 2.19 |
| oculocutaneous albinism II (pink-eye dilution homolog, mouse) | 2.16 |
| Inhibin, beta A (activin A, activin AB alpha polypeptide) | 2.16 |
| Allograft inflammatory factor 1 | 2.12 |
| Isocitrate dehydrogenase 2 (NADP+), mitochondrial | 2.10 |
| Collagen, type III, alpha 1 (Ehlers-Danlos syndrome type IV, autosomal dominant) | 2.07 |
| tenascin C | 2.07 |
| procollagen-proline, 2-oxoglutarate 4-dioxygenase (proline 4-hydroxylase), alpha | 2.06 |
| Polymerase (RNA) II (DNA directed) polypeptide B, 140kDa | 2.06 |
| Cathepsin L | 2.05 |
| lipoprotein lipase | 2.05 |
| Tumor-associated calcium signal transducer 1 | 2.05 |
| acyl-CoA synthetase long-chain family member 4 | 2.03 |
| scavenger receptor for phosphatidylserine and oxidized low density lipoprotein | 2.03 |
| cyclin-dependent kinase inhibitor 3 | 2.01 |
| calponin 1, basic, smooth muscle | 2.01 |
| Occludin | 2.01 |
| Gelsolin | 2.00 |
| bestrophin 1 | 1.98 |
| laminin, beta 1 | 1.97 |
| proteasome (prosome, macropain) subunit, beta type, 10 | 1.97 |
| transcription factor PU.1 | 1.95 |
| DNase X | 1.95 |
| dopa decarboxylase | 1.95 |
| propionyl Coenzyme A carboxylase, beta polypeptide | 1.94 |
| glutaminase | 1.92 |
| Chromosome 17 clone pkmCon20, mRNA sequence | 1.91 |
| occludin | 1.91 |
| Histone H1.3-like protein | 1.88 |
| cathepsin D | 1.87 |
| Coxsackie-adenovirus-receptor homolog | 1.86 |
| vascular endothelial growth factor | 1.85 |
| Tropomodulin 3 (ubiquitous) | 1.84 |
| pyridoxal (pyridoxine, vitamin B6) kinase | 1.83 |
| 38 kDa heparin-binding glycoprotein | 1.83 |
| 2',5'-oligoadenylate synthetase 1, 40/46kDa | 1.82 |
| tissue inhibitor of metalloproteinase-3 | 1.82 |
| calcium channel, voltage-dependent, beta 4 subunit | 1.82 |
| aminoacylase 1 | 1.80 |
| Heat shock protein 47 | 1.80 |
| fatty acid binding protein 7, brain | 1.80 |
| interleukin 6 (interferon, beta 2) | 1.80 |

| **DOWNREGULATED GENES** | |
|---|---|
| **Description** | **Fold Change** |
| Estrogen sulfotransferase | -4.03 |
| tumor-associated calcium signal transducer 1 | -3.59 |
| alveolar macrophage-derived chemotactic factor-I | -3.14 |
| porcine inhibitor of carbonic anhydrase | -2.71 |
| carboxypeptidase A1 precursor | -2.50 |
| Fibrinogen-like 2 | -2.48 |
| v-myc myelocytomatosis viral oncogene homolog (avian) | -2.47 |
| Polo-like kinase 2 (Drosophila) | -2.46 |
| Endogenous retrovirus Tsukuba-1 mRNA, complete sequence | -2.46 |
| fibrinogen-like 2 | -2.41 |
| phosphogluconate dehydrogenase | -2.41 |
| Alpha-2-macroglobulin | -2.37 |
| fibrinogen-like 2 | -2.35 |
| glutamine-fructose-6-phosphate transaminase 1 | -2.33 |
| interleukin 13 receptor, alpha 1 | -2.32 |
| lysozyme | -2.14 |
| fibrinogen-like 2 | -2.11 |
| similar to tigger transposable element derived 4 | -1.99 |
| glutamine-fructose-6-phosphate transaminase 1 | -1.97 |
| CD74 antigen | -1.97 |
| metallothionein isoform | -1.92 |
| Interleukin 13 receptor, alpha 1 | -1.92 |
| complement component 1, q subcomponent, B chain | -1.90 |
| antileukoproteinase | -1.90 |
| stathmin-1 | -1.89 |
| high-affinity copper uptake protein | -1.89 |
| insulin-like growth factor 2 (somatomedin A) | -1.89 |
| lactotransferrin | -1.89 |
| Antithrombin III | -1.87 |
| solute carrier family 7 (cationic amino acid transporter, y+ system), member 1 | -1.82 |

Table 1 shows the fold changes in genes from capsuloids grown for 3 days in RPMI-CPN media compared to CP clusters grown in the same media, and Table 2 shows the fold changes in genes in CP clusters grown for 3 days in ENDO-SFM media compared to CP clusters grown in RPMI-CPN media. Table 3 below highlights some of the genes upregulated in the presence of ENDO-SFM that are implicated in wound healing.

**TABLE 3**

| **GENE EXPRESSION LEVEL CHANGES IN ENDO-SFM:** | |
|---|---|
| **Upregulated Primary Regenerative Candidates** | |
| **Gene with Wound Healing Potential** | **Fold Change in ENDO-SFM** |
| Haptocorrin | +43.16 |
| Tropomodulin 3 | +8.37 |
| Matrix GLA Protein | +5.30 |
| Type III Collagen | +5.22 |
| PEDF | +4.55 |
| β-2-microglobulin | +4.20 |
| FGF-2 | +3.47 |
| Type I Collagen | +3.16 |
| Somatostatin | +2.94 |
| Fibronectin | +2.80 |
| TGF-β | +2.25 |
| Laminin β1 | +1.97 |
| VEGF | +1.85 |
| I1-6 | +1.80 |

### Example 9: Nanoencapsulation of lyophilised choroid plexus conditioned media and demonstration of protein integrity as assessed by ELISA

Poly(lactide-co-glycolide) was used to encapsulate micronised, lyophilised Choroid Plexus conditioned media (LCPCM). LCPCM described previously was reconstituted in a super saturated solution of distilled water (236.72 ing in 2 mL). Separately, 25 mL ethyl acetate was added to 413 mg PLGA (Resomer® RG503H, Boehringer Ingelheim), a biodegradable polyster with 50:50 copolymer ratio and a weight-average molecular weight of 9 kDa, considered relatively fast-degrading. The two solutions were vortexed vigorously and probe-sonicated to create a microemulsion, which was rapidly frozen in liquid nitrogen and subsequently lyophilised. Due to the poor miscibility of ethyl acetate and water in combination with the aggressive mixing techniques employed, very small droplets of CPCM were frozen with the lipophilic polymer-chloroform matrix. After lyophilisation to complete dryness, this yielded a solid matrix of PLGA surrounding micronised particulates of lyophilised conditioned medium.

This micronisation technique was employed to improve the homogeneity of the formulation, to reduce the overall size of the microspheres, and to enhance solubility due to the larger surface area to volume ratio of the resulting therapeutic particulates. This polymer-CPCM combination was then dissolved in 40 mL ethyl acetate, which thoroughly dissolved the PLGA component but retained the micronised CPCM as insoluble microparticlates. The solution was rapidly dispersed into 2 L heptane, causing rapid precipitation of polymer around the micronised CPCM. The resultant therapeutic microspheres consisted of 36.5% (W/W) loading of CPCM, or roughly 127 pg VEGF/mg microsphere formulation.

Microspheres were characterised using scanning electron microscopy for surface morphology, Coulter particle size analysis for size distribution, and organic extraction to determine if one of the CPCM proteins, vascular endothelial growth factor (VEGF) remained detectable and potentially active following exposure to the solvents employed.

Extraction of VEGF was accomplished by dissolving 20 mg microspheres in ethyl acetate, extracting proteins into an aqueous buffer, and measuring VEGF using an ELISA. In this manner, it was determined that 20mg microspheres contained 1,509 +/- 65 pg VEGF, or 75.5 pg/mg microspheres. This correlates with 40% VEGF losses due to processing or inefficient extraction.

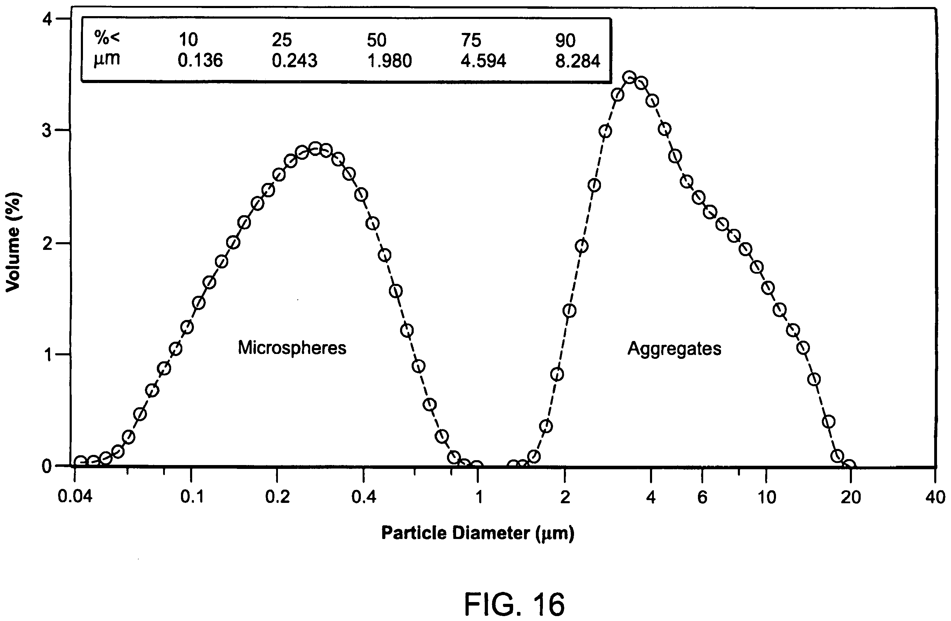

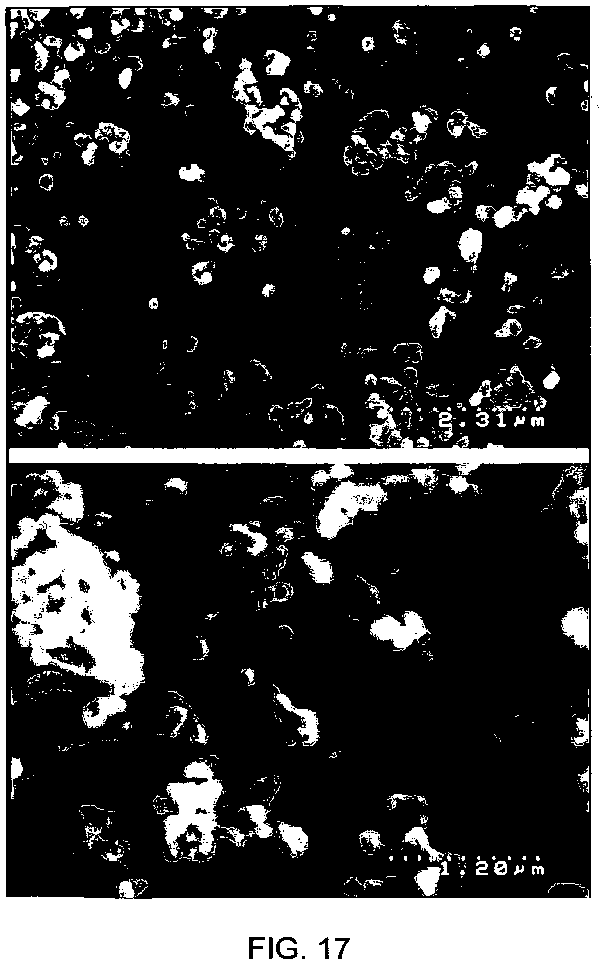

Coulter particle size analysis and scanning electron microscopy revealed that microspheres were roughly 300 nm in size, with aggregation occurring within the dispersion to create clusters with mean diameters of approximately 4 and 10 microns respectively, (Figure 16 and 17).

### Example 10: Analysis of the Transcriptome of CP in cultures generating condition media

Microarray analysis of the cells secreting the CP conditioned lyophilised media produced using ENDO-SFM media, as described in example 8, above, was carried out and the level of expression of secreted proteins measured. Of the over 24,000 expression values returned from the porcine microarray, some genes involved in the wound healing process are summarized below in Table 4. Each of the secreted proteins encoded by these genes has been implicated in the steps that make up the wound healing process.

**TABLE 4**

| **GENE** | **Log₂ Expression Values (ENDO-SFM)** | **Angiogenic** | **Migration and Proliferation** | **Trophic** | **Tissue Remodeling** | **Inflammation** |
|---|---|---|---|---|---|---|
| Transthyretin | 15.12 +/- 0.03 | | | ✔ | | |
| Ubiquitin C | 14.67 +/- 0.01 | | | ✔ | | |
| Clusterin | 14.15 +/- 0.04 | ✔ | | | | |
| Connective Tissue Growth Factor | 14.01 +/- 0.01 | | | ✔ | | |
| Osteonectin | 14.01 +/- 0.06 | ✔ | ✔ | | | |
| Beta-2-microglobulin | 13.98 +/- 0.02 | | | | ✔ | |
| Insulin-like Growth Factor Binding | 13.65 +/- 0.01 | | | | ✔ | |
| Protein | | | | | | |
| Cystatin C | 13.31 +/- 0.04 | | | | | ✔ |
| Aquaporin 1 | 13.30 +/- 0.01 | | ✔ | | | |
| Optineurin | 12.73 +/- 0.03 | | | ✔ | | |
| Neurolysin | 12.70 +/- 0.01 | ✔ | | | ✔ | |
| COFILIN protein | 12.60 +/- 0.01 | | | | ✔ | |
| Secreted Folate Binding Protein | 12.59 +/- 0.04 | | ✔ | | | |
| Growth Associated Protein 43 | 12.50 +/- 0.01 | | ✔ | | | |
| Cellular Retinol Binding Protein 1 | 12:45 +/- 0.01 | | ✔ | | ✔ | |
| TIMP metallopeptida se inhibitor 1 | 12.40 +/- 0.01 | | | | ✔ | |
| MIF2 suppressor | 12.28 +/- 0.03 | | | | | ✔ |
| Neuronal Protein 3 | 12.27 +/- 0.01 | | | ✔ | | |
| TIMP-2 | 12.01 +/- 0.10 | | | | ✔ | |
| TrkB Protein | 12.01 +/- 0.01 | | | ✔ | | |
| Thioredoxin Interacting Protein | 11.93 +/- 0.06 | | | | ✔ | |
| Osteoclast Stimulating Factor | 11.82 +/- 0.03 | | | ✔ | | |
| Insulin induced gene 1 | 11.78 +/- 0.04 | | | ✔ | | |
| Vascular endothelial growth factor | 11.66 +/- 0.03 | ✔ | | | | |
| Collagen III | 11.47 +/- 0.01 | | | | ✔ | |
| Interferon Regulatory Factor 3 | 11.41 +/- 0.05 | ✔ | | | | |
| Transforming Growth Factor | 11.39 +/- 0.07 | | ✔ | | ✔ | ✔ |
| Beta 2 | | | | | | |
| Insulin-like growth factor 2 | 11.26 +/- 0.04 | ✔ | ✔ | ✔ | | |
| Fibromodulin | 11.19 +/- 0.03 | | | | ✔ | |
| Pro-melanin concentrating hormone | 11.11 +/- 0.06 | | | | ✔ | |
| Pigment epithelium-derived factor | 11.07 +/- 0.01 | ✔ | | ✔ | | |
| Parathyroid hormone-like hormone | 10.97 +/- 0.03 | | ✔ | | | |
| Bone morphogenetic protein 7 | 10.96 +/- 0.01 | | | | ✔ | |
| Antithrombin III | 10.87 +/- 0.01 | | ✔ | | | |
| Neuronal endocrine protein | 10.85 +/- 0.04 | | ✔ | | | ✔ |
| Metallothionei n-III | 10.85 +/- 0.03 | | ✔ | | | |
| Caveolin 1 | 10.79 +/- 0.06 | | ✔ | | | |
| CXCL-14 Chemokine | 10.79 +/- 0.05 | ✔ | | | | |
| Smooth Muscle Protein 22-alpha | 10.49 +/- 0.01 | | ✔ | | | |
| Monoamine Oxidase B | 10.64 +/- 0.05 | | ✔ | | | |
| Matricin | 10.49 +/- 0.02 | | | | | ✔ |
| ICAM-1 | 10.27 +/- 0.09 | | | | ✔ | |
| Somatostatin | 10.03 +/- 0.03 | | ✔ | | | |
| Calpain, small subunit 1 | 10.14 +/- 0.01 | | | | ✔ | |
| Fibronectin | 9.94 +/- 0.02 | | | | ✔ | |
| Adrenomedulli n | 9.75 +/- 0.02 | ✔ | ✔ | ✔ | | |
| Interleukin 6 | 9.67 +/- 0.07 | | ✔ | | ✔ | |
| Axotrophin | 9.66 +/- 0.08 | ✔ | | | ✔ | |
| Connexin 43 | 9.50 +/- 0.03 | | ✔ | | | |
| Tissue Factor | 9.50 +/- 0.01 | ✔ | | | | |
| Collagen Type VIII | 9.08 +/- 0.10 | | | | ✔ | |
| Interleukin 1 | 8.82 +/- 011 | | ✔ | ✔ | | |
| Transforming Growth Factor Alpha | 8.51 +/- 0.05 | | ✔ | | | ✔ |
| Integrin Alpha V | 8.27 +/- 0.13 | | | | ✔ | |
| Interleukin 15 | 8.26 +/- 0.05 | | | ✔ | | |
| Collagen Type V | 8.19 +/- 0.07 | | | | ✔ | |
| Vascular Cell Adhesion Molecule | 8.10 +/- 0.11 | ✔ | | | ✔ | |
| Monocyte Chemoattracta nt Protein 1 | 7.29 +/- 0.05 | | | | | ✔ |

### Example 11: Endothelial Tube Formation In Choroid Plexus Co-Cultures

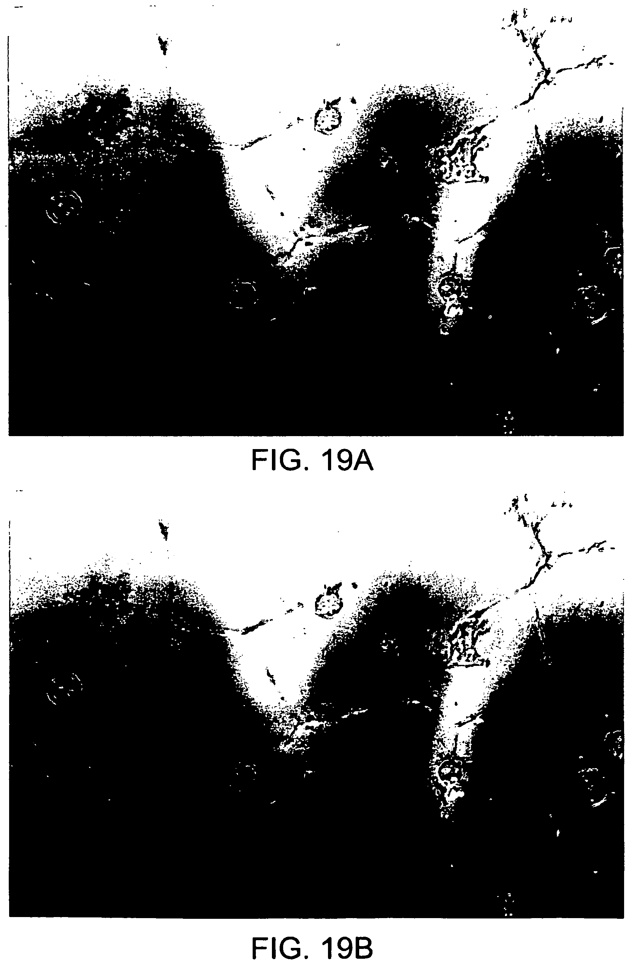

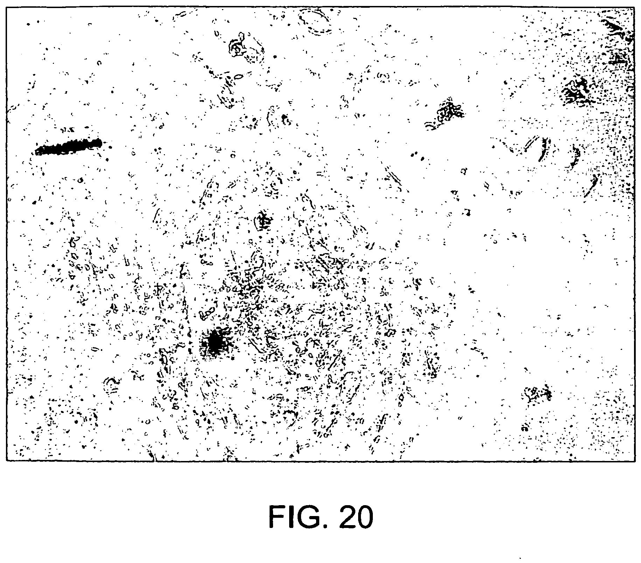

Choroid plexus epithelial clusters isolated from Yorkshire swine were incubated in a growth-factor rich three-dimensional hydrogel for 7 days in RPMI 1640 tissue culture medium containing 2% fetal bovine serum (2500 clusters / cm²). The morphology of the clusters, as described elsewhere, transitioned from a solid spheroidal cell mass to a hollow capsuloid. Separately, human umbilical vascular endothelial cells (HUVEC, Invitrogen) were grown to 95% confluency in t75 flasks in Medium 200 (Invitrogen). The medium was changed daily. HUVEC were then trypsinized and added to the wells of 3-D choroid plexus cultures at a density of 2.5 x 10³ cells / cm²) in RPMI 1640 / FBS, or to wells containing the matrix alone. After 24 hours of incubation, HUVEC co-cultured with CP capsuloids demonstrated a change in phenotype from ovoid, epithelial cells to tubular structures as shown in Figure 19A and 19B below. Importantly, the network formed by the endothelial tubes appears to be guided by the placement of CP capsuloids, as nearly each cell mass serves as a node in the vascular network. In contrast, HUVEC cultured under the same conditions but without choroid plexus remained undifferentiated (Figure 20). This behavior is indicative of exposure to an angiogenic stimulus, which is secreted in the cocktail of growth factors provided by the choroid plexus.

### Example 12: CP Isolation and Culture

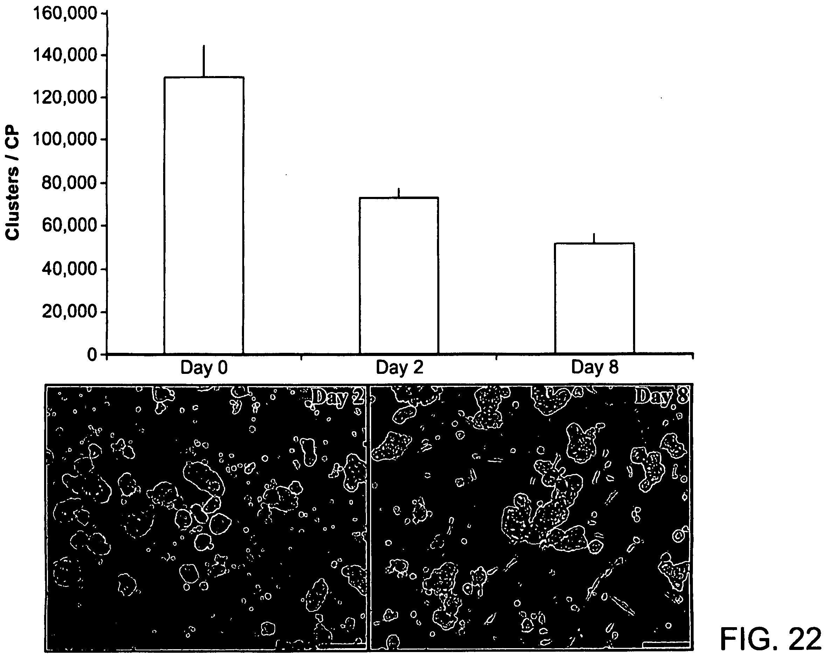

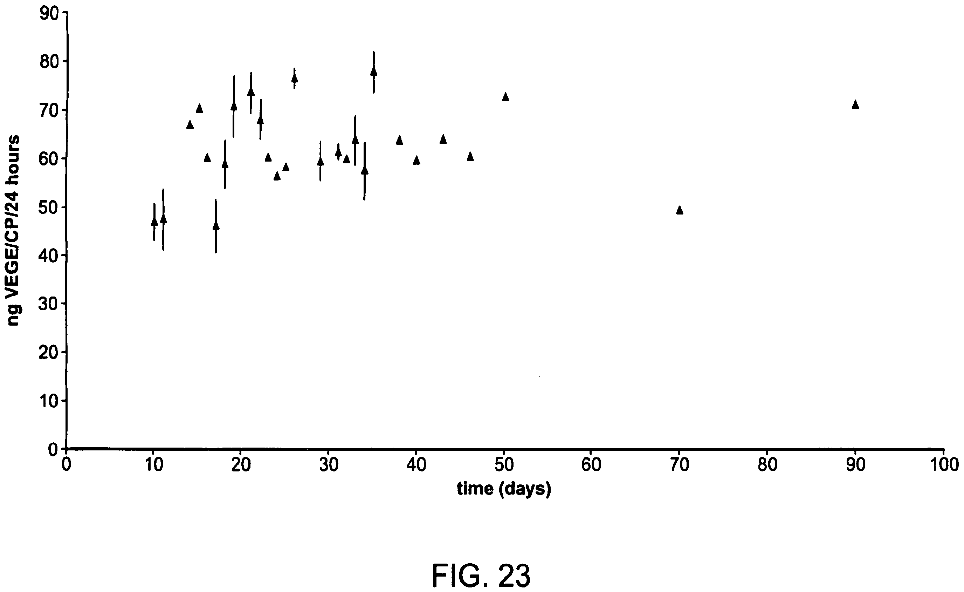

CP was successfully isolated from neonatal pigs and digested into CP clusters ranging in size from approximately 50-250 µm. After culturing the clusters in non-adherent flasks in RPMI-CPN for 8 days, manual counting revealed that the yield per piglet decreased from 128,854 ± 16,159 to 50,962 ± 5,690 CP clusters. This is consistent with the temporal loss of non-epithelial clusters of cells due to attachment or death, in addition to gradual coalescence of small aggregates into larger clusters (Figure 22). After the transition to serum-free media after 10 days, CM was collected in parallel with fresh medium held under the same conditions. VEGF levels were measured in aliquots taken from CM generated throughout the experiment (Figure 23) as well as control medium. 10 days after isolation, daily levels of VEGF secreted per CP increased from approximately 41 ng to around 70 ng at 2 weeks, and remained relatively stable thereafter. Following processing of the conditioned medium to generate a pooled purified lyophilate, VEGF levels were verified to ensure that the molecule was conserved throughout the process.

### Example 13: CP Gene Array

CP cohorts from the same animal group were cultured, after 10 days in RPMI-CPN, in ENDO-SFM for an additional 3 or 16 days specifically for microarray analysis. Whole genome porcine arrays were performed to characterize the CP expression pattern as well as to evaluate the plasticity of the CP transcriptome over the first 16 days following media transition. The microarray data were selected and sorted based on the potential of mRNAs to transcribe for secreted molecules, relevance in tissue regeneration, expression levels, and statistical significance. Shown in Table 5 is a list of those factors that could play a role in wound healing, along with a descriptive matrix of their potential roles. As expected (Thanos, C. G.; et al., The in vitro expression and secretion of vascular endothelial growth factor from free and alginate-polyomithine encapsulated choroid plexus epithelium. Tissue Eng. 13:747-756; 2007; Thouvenot, E.; et al. The proteomic analysis of mouse choroid plexus secretome reveals a high protein secretion capacity of choroidal epithelial cells. Proteomics 6:5941-5952; 2006), transthyretin was expressed at extremely high levels in tissue culture, and a number of other factors were expressed at similarly high levels. In general, the regenerative genes listed encode for growth factors, angiogenic factors, extracellular matrix remodeling components, and other factors with regenerative properties. The regenerative factor compliment expressed by the CP clusters in ENDO-SFM was significant, and included several relevant factors (see table for abbreviations): CTGF (14.01), VEGF (11.66), TGF-β (11.39), IGF-2 (11.26), PEDF (11.07), BMP-7 (10.96), PDGF (10.59), Somatostatin (10.03), I1-6 (9.67), Tissue Factor (9.50), WISP-1 (8.85), FGF-2 (8.79), TGF-α (8.51), Wnt-10B (8.27), EGF (7.57) and others. The difference in expression levels of most of these regenerative components between day 3 and 16 was nearly indistinguishable, with most factors remaining within a very narrow range, except for CXCL-14 chemokine and adrenomedullin, which decreased by 3.58-fold and 2.08-fold respectively. The entire array database was subsequently sorted to compare statistically significant changes greater than 1.8-fold in all expression levels between day 3 and day 16, the output of which is shown in Table 6. These genes that exhibit the highest variability in temporal expression could be involved in the acclimation of CP to tissue culture, and do not include major regenerative candidates.

### Example 14: Monolayer Scratch Assay

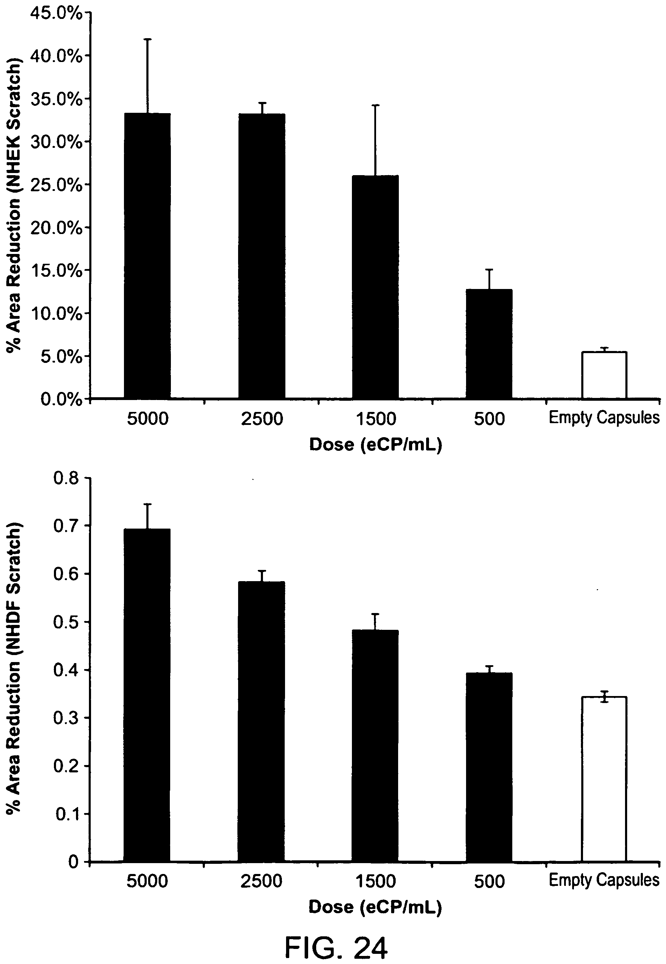

Cultured monolayers of neonatal human keratinocytes and fibroblasts were disrupted and incubated with different doses of encapsulated CP in basal culture medium. In both cell types, the wounded area decreased during the incubation period and was significantly smaller in groups treated with > 1500 encapsulated CP. The data, shown in Figure 24, demonstrate that the chemotaxic effect was dose-dependent and the peak effect was associated with a 35.2% area reduction over control in NHDF cultures, and a 27.9% area reduction over control in the NHEK cultures. In NHEK cultures, 2500 encapsulated CP was associated with the smallest wound area, while the NHDF cells showed an increased response up to the maximum dose of 5000 encapsulated CP. To gauge the levels of VEGF secreted by the encapsulated CP, samples of 2500 (N=9) were assessed as described (Thanos, C. G.; et al., The in vitro expression and secretion of vascular endothelial growth factor from free and alginate-polyornithine encapsulated choroid plexus epithelium. Tissue Eng. 13:747-756; 2007). At the dose of 2500 encapsulated CP / well, roughly 2.4 ng VEGF was secreted over 24 hours.

### Example 15: Full Thickness Open Wounds

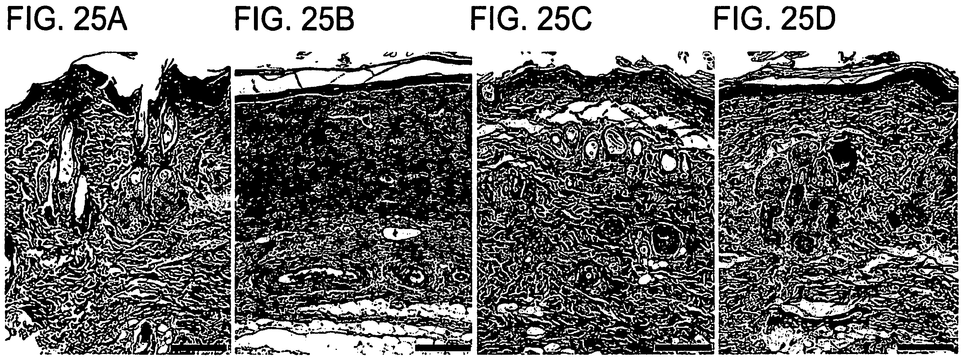

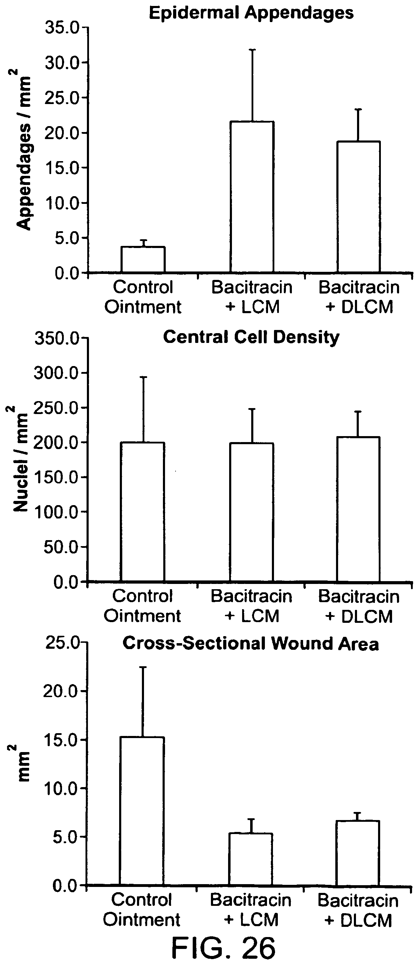

Uncovered open wounds were treated daily for 10 days with no sign of infection. By day 3, scabs formed on all wounds with no obvious difference between groups; after 8 days, the scabs began to detach as the underlying wounds contracted. After 2 weeks, wound surfaces appeared relatively smooth with little evidence of scab remnants. Superficially, all wounds looked well healed by this time point, with some variability in the presence of hair follicles evident in the central wounded area. Figure 21 shows explanted skin containing the wounded area, with a control treatment (Figure 21 A) and LCM treatment (Figure 21B), which contains more follicles within the wounded area. This observation is more apparent histologically, as shown in Figure 25. Here, a series of representative sections are shown located 1/3 into the width of the wound. In Figure 25A, normal skin is shown, which contains epidermal appendages including hair follicles associated with sebaceous glands, dense collagen, and distinctly formed epidermal rete pegs. By comparison, a wound treated with bacitracin alone, shown in Figure 25B, contains densely packed fibroblasts within the dermis, replete with newly formed vessels, and an epidermis of a thin, flat layer of keratinocytes. Wounds treated with LCM (Figure 25C), contain tissue with a higher level of organization, containing forming epidermal appendages, a lower cell density, and a more ridged profile suggestive of epidermal rete pegs. Similarly, DLCM-treated wounds (Figure 25D) were rich in epidermal appendages, including sebaceous glands, which occasionally extended through the epidermis manifesting as visible hair. These wounds also resembled the morphology of normal tissue in the density and ratio of cells to collagen. As shown in Figure 26, wounds treated with CP products contained a significantly higher density of epidermal appendages (5- and 5.8-fold higher for DLCM and LCM respectively) and an overall smaller cross-sectional area. However, central cell density was morphometrically indistinguishable between groups.

### Linear Incisions

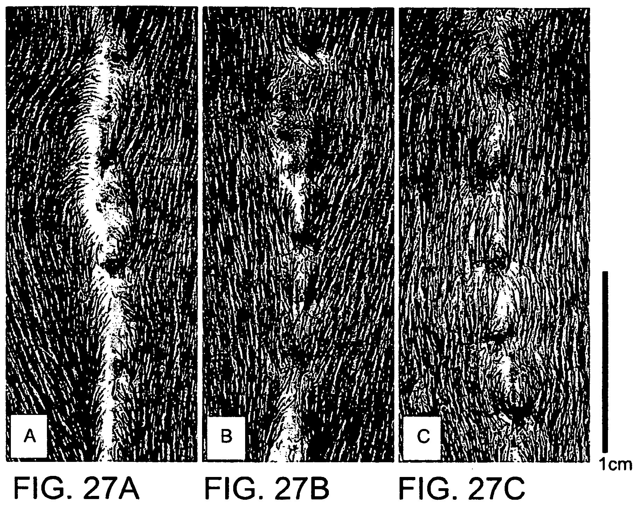

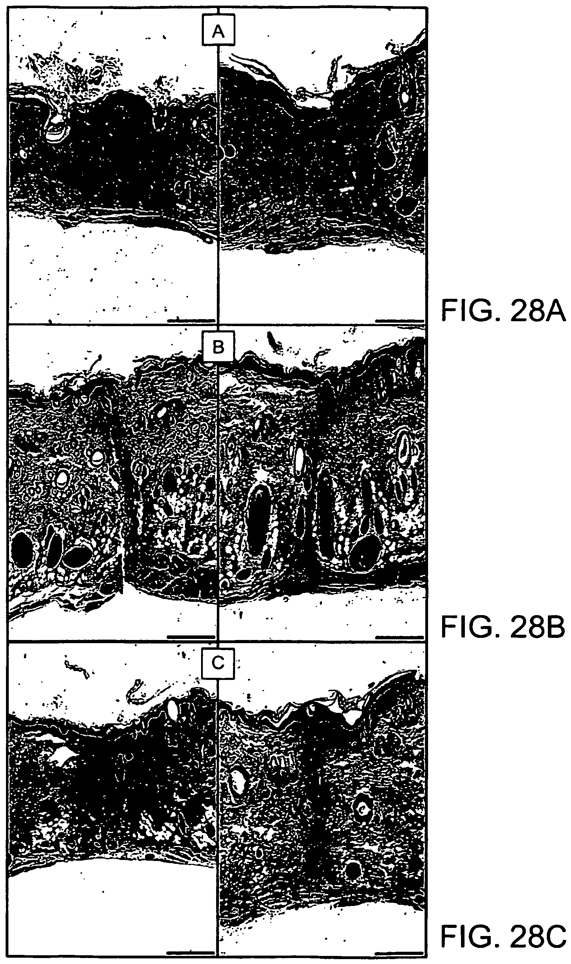

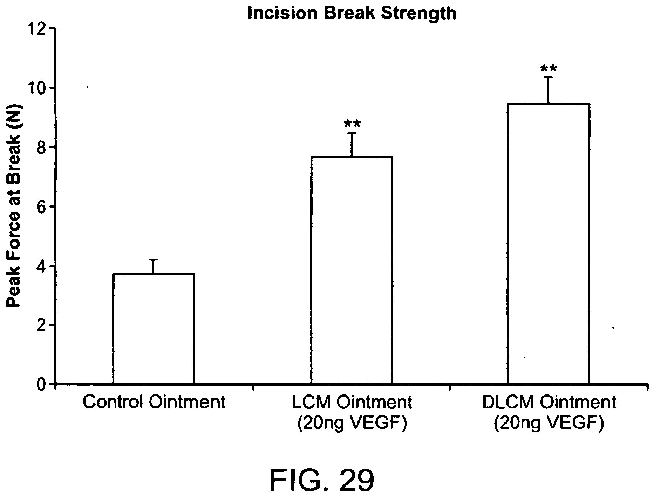

6-cm incisions were treated topically with ointments for 10 days prior to skin harvest 4 days later, at day 14. All wounds healed completely during the study, with complete closure occurring after 8 days (Figure 27). Incisions treated with CP-proteins showed a marked improvement in the cosmetics of the incisions, with LCM (Figure 27B) and DLCM (Figure 27C) appearing more symmetric, with narrower margins than the control group (Figure 27A). This finding was consistent with the histology, which showed much thinner bands of fibrosis in the center of the wound, along with a greater density of epidermal appendages at the margins (Figure 28). The control group (Figure 28A) had a large area of fibrosis in the healing incision, while both LCM (Figure 28B) and DLCM (Figure 28C) reduced this area dramatically. Interestingly, LCM demonstrated the strongest effects, with the width of the central scar reduced roughly 2-fold over the DLCM treatment, and roughly 5-fold over the control group. As demonstrated with end-to-end tensiometry, this improved cosmetic performance carried over to the strength of the tissue. As shown in Figure 29, both groups treated with CP proteins were associated with a statistically significant improvement in peak break strength over the control. LCM increased strength over the control group from 3.7 +/- 0.5 N to 7.7 +/- 0.8 N, while DLCM was associated with a further increase in force to 9.5 +/- 1.6 N.

### Discussion

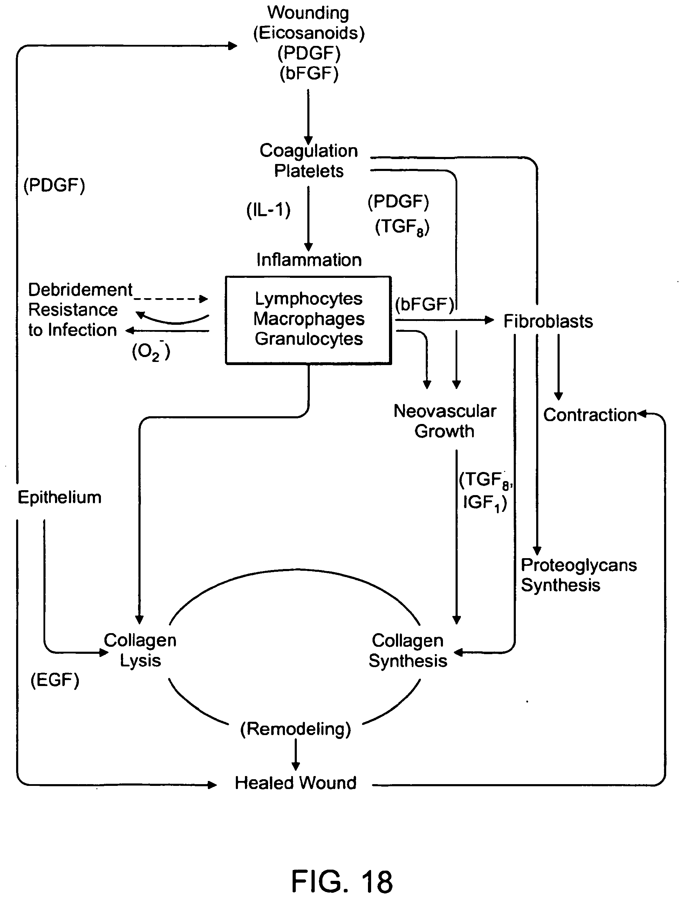

The CP secretes proteins useful in maintaining the health and function of the brain and CNS. Such secreted proteins can help in treating neurological diseases. Surprisingly, the current results demonstrate that CP secreted proteins are also involved in regulating the inflammatory response and in improving wound healing and reducing scar tissue formation. As part of the normal wound healing process macrophages infiltrate the wound site soon after wounding and by release of chemokines contribute to key processes in healing such as regulation of epitheliasation, tissue remodeling and angiogenesis in skin¹¹ and other tissues. A schematic diagram of the wound healing process is set out in Figure 18.

In the present examples, histological data demonstrates that there is increased and accelerated infiltration of macrophages and fibroblasts into the wound area resulting in the laying down of fibrotic scar tissue (Figures 7b and 7c - control). In CP secreted protein treated wounds, the accumulation of fibrotic connective tissue is reduced (Figure 7b and 7c - treated) and tissue function maintained as evidenced by improved break strength (Figure 8). During wound healing the injured necrotic area is invaded by small blood vessels, mononuclear cells and activated macrophages. These activated lymphocytes simultaneously secrete several cytokines and growth factors, which are critical in chemotaxis and subsequent wound healing processes. CP secreted proteins also comprise numerous cytokines and growth factors and appear to accelerate tissue repair by enhancing the wound healing process leading to accelerated healing. Perhaps this is due to a synergistic effect of the endogenous growth factors and the exogenous growth factors supplied by the administration of CP secreted proteins. The result seen in the present examples is not due to the presence of VEGF alone. A composition comprising CP secreted proteins was more effective at accelerating wound healing than recombinant VEGF alone (results not shown).

Our results show that lyophilized CP conditioned media is a potent stimulator of the wound healing process resulting in increased wound strength and reduced wound scarring.

Fibrosis is a part of the wound healing processes but excess fibrosis leads to scarring and reduced function of tissues. Fibroblasts play a major role in deposition of collagen and thus scar formation in wounds. Studies have previously correlated the extent of fibroblast accumulation with scarring in skin burn wounds¹⁰. It is anticipated that CP secreted proteins acts to reduce scarring by decreasing the accumulation of fibroblasts at a wound site with a consequent decrease in scarring in the healed wound. Importantly, the data presented here shows that the administration of CP secreted proteins to a wound site in vivo leads to decreased collagen accumulation and scarring in tissue that has undergone wound healing. Evidence for this is already available in example 7 above and figure 9. Collagen has been found to be the major pathological finding in a number of fibrotic diseases¹², but at the same time is an extremely important early component of non-fibrotic tissue regeneration. This fine balance of extracellular matrix production, chemotaxis, and mitogenesis in wound healing therapy can only be achieved by concomitant or staged delivery of a multitude of individual factor therapies, whereas in this work it is apparent that the CP-derived cocktail provides all aspects.

The studies presented here demonstrate the ability of factors secreted by the CP to intervene in the wound healing process. In vitro, this activity was manifested by encapsulated CP co-cultured with scratched monolayers cultures of human fibroblasts and keratinocytes as an accelerated wound area reduction due to chemotaxis or cell proliferation. In these models, there was an increase in the amount of healing associated with the dose of VEGF in the conditioned medium. In vivo, open wounds treated with CPCM in ointment showed a 5- to 6-fold increase in replenishment of epidermal appendages after 14 days compared to the control, along with a significant reduction in cross-sectional wound area. And surgically closed incisions treated with CPCM were associated with a greater than 2-fold increase in the tensile strength of the healed tissue, and an overall better cosmetic appearance with reduced central scarring and better preservation and / or repopulation of epidermal appendages surrounding the wound margins.

While the precise healing mechanism associated with CP proteins remains unclear, without wishing to be bound by theory, it is likely related to the synergistic activity of a combination of expressed factors. The transcriptome of CP contains a number of genes encoding for mediators of the natural wound healing response. While it is unknown if the full compliment of these factors is secreted from CP in the tissue culture system described here, proteomic analysis of mouse CP supports an abundance of secreted proteins (Thouvenot, E.; et al. The proteomic analysis of mouse choroid plexus secretome reveals a high protein secretion capacity of choroidal epithelial cells. Proteomics 6:5941-5952; 2006). VEGF, chosen as a surrogate marker of potency in these studies, was delivered at biologically efficacious levels (Ozawa, C. R.; et al. Microenvironmental VEGF concentration, not total dose, determines a threshold between normal and aberrant angiogenesis. J. Clin. Invest. 113:516-527; 2004), and is capable of up-regulating intrinsic factors such as PDGF (Galiano, R. D.; et al. Microenvironmental VEGF concentration, not total dose, determines a threshold between normal and aberrant angiogenesis. Am. J. Pathol. 164:1935-1947; 2004), bFGF (Galiano, R. D.; et al. Microenvironmental VEGF concentration, not total dose, determines a threshold between normal and aberrant angiogenesis. Am. J. Pathol. 164:1935-1947; 2004), TGF- β1 (Li, Z.-D.; et al. VEGF induces proliferation, migration, and TGF-β1 expression in mouse glomerular endothelial cells via mitogen-activated protein kinase and phosphatidylinositol 3-kinase. Biochem. Biophys. Res. Commun. 334:1049-1060; 2005), and others that are involved in wound healing. Still, it is likely that the overall effect of the CP cocktail extends beyond VEGF alone, as there are a number of other highly expressed factors contained within the CP transcriptome that act at similarly high levels.

CTGF, for example, is expressed by CP at levels nearly as high as transthyretin, the factor with the highest expression level in gene array (Thanos, C. G.; et al., The in vitro expression and secretion of vascular endothelial growth factor from free and alginate-polyomithine encapsulated choroid plexus epithelium. Tissue Eng. 13:747-756; 2007) and proteomic analysis (Thouvenot, E.; et al. The proteomic analysis of mouse choroid plexus secretome reveals a high protein secretion capacity of choroidal epithelial cells. Proteomics 6:5941-5952; 2006). CTGF promotes chemotaxis and proliferation of fibroblasts, the formation of granulation tissue, re-epithelialization, and matrix remodeling (Igarashi, A.; et al. Regulation of connective tissue growth factor gene expression in human skin fibroblasts and during wound repair. Mol. Biol. Cell 4:637-645; 1993). While the primary role of CTGF in wound healing is to promote tissue reconstruction through fibrosis, it has also been shown to regulate VEGF-mediated angiogenesis through negative feedback (Jang, H. S.; et al. A novel ex vivo angiogenesis assay based on electroporation-mediated delivery of naked plasmid DNA to skeletal muscle. Mol. Ther. 9:464-474; 2004), and is angiogenic on its own in models of corneal neovascularization (Shimo, T.; et al. Connective tissue growth factor induces the proliferation, migration, and tube formation of vascular endothelial cells in vitro, and angiogenesis in vivo. J. Biochem. 126:137-145; 1999). TGF-β, also highly expressed by the CP, is one of the most well known mediators of cutaneous wound healing, and is involved from the inflammatory phase through tissue remodeling and re-epithelialization (Barrientos, S.; et al. Growth factors and cytokines in wound healing. Wound Repair Regen. 16:585-601; 2008). It has demonstrated therapeutic potential in topical application to venous stasis ulcers (Robson, M. C.; et al. Safety and effect of transforming growth factor-beta(2) for treatment of venous stasis ulcers. Wound Repair Regen. 3:157-167; 1995), and has been used to increase the strength of incisional wounds (Wright, T. E.; et al. The effect of TGF-beta2 in various vehicles on incisional wound healing. Int. J. Surg. Investig. 2:133-143; 2000). IGF-2, BMP-7, PDGF, FGF-2, EGF, and other factors expressed by CP and listed in Table 5 also have potential wound healing effects as cited in the literature and as indicated in the table.

One of the most intriguing observations in these studies is the increased density of hair follicles within the wound margin following CP factor administration. While it is unclear if the follicles were generated de novo in both models, it is likely that follicles present in the healed open wounds were a result of neogenesis. These follicles, varying in their maturity but appearing in healing tissue prior to 2-weeks, may have developed as a result of Wnt-dependent signaling involving Wnt10b, which is expressed at moderate levels by CP. In vivo, Wnt10b is important in the differentiation of skin epithelium and formation of hair follicles (Ito, M.; et al. Wnt-dependent de novo hair follicle regeneration in adult mouse skin after wounding. Nature 447:316-320; 2007), and recent work using recombinant Wnt10b has demonstrated positive effects on hair shaft growth and follicle formation in vitro (Ouji, Y.; et al. Wnt-10b, uniquely amount Wnts, promotes epithelial differentiation and shaft growth. Biochem. Biophys. Res. Commun. 367:299-304; 2008). Perhaps as a component of the CP cocktail, Wnt10b works in concert with factors that are involved in the earlier stages of wound healing, e.g. PDGF, VEGF, CTGF, TGF- β, and others, to guide epithelial differentiation concurrent with angiogenesis and migration of fibroblasts and keratinocytes, effectively providing developmental cues as in embryogenesis.

Still, the effects observed in these studies support the role of therapeutic CP-products outside of the central nervous system. They are a ready source of various factors that, when applied as individual molecules, are associated with improvements in wound healing. Recombinant PDGF, for example, has been studied extensively on its own to treat diabetic ulcers (Robson, M. C.; et al. Platelet-derived growth factor BB for the treatment of chronic pressure ulcers. Lancet 339:23; 1992), and is approved by the FDA for topical use. TGF-β2 has also been tested clinically (Robson, M. C.; et al. Effects of Transforming Growth Factor B2 on Wound Healing in Diabetic Foot Ulcers: A Randomized Controlled Safety and Dose-Ranging Trial. JARCET 2(2); 2002), and remains a fertile area of product development. The application of these and other factors simultaneously, to be used as required by the healing tissue, would address issues related to targeting just one aspect of the wound healing cascade, and decrease the impact of delivery kinetics and the regulation of factors via feedback in the wound. Such a combination of factors could also prove useful in more severe wounds, such as deep ischemic ulcers, that would require a more coordinated rebuilding of tissue beginning with a restoration of blood supply and ending with superficial reconstruction.

Factors from the CP, delivered in a biologically determined ratio and composition as shown here, could be used to improve the healing of cutaneous wounds. Because of the potentially widespread effect imparted by the CP cocktail, ranging from angiogenesis to tissue rebuilding, there may be other indications that are also suitable for investigation. A therapeutic benefit has already been described in animal models of neurodegenerative disease, and other potential indications include diseases where staged intervention is required to treat tissues suffering from ischemia and necrosis, such as diseases of the cardiovascular system, muscle wasting and repair, hair regrowth, antiwrinkle treatment, cartilage repair, and other similar areas.

### Conclusion

Therapeutic compositions comprising CP secreted proteins, have been shown here to increase the rate of wound healing by acceleration and enhancement of several key processes. The application of CP secreted proteins has also been shown to result in decreased deposition of collagen at the final healed wound site which prevents loss of tissue function (strength) or cosmetic damage due to scarring.

It is not the intention to limit the scope of the invention to the abovementioned examples only. As would be appreciated by a skilled person in the art, many variations are possible without departing from the scope of the invention (as set out in the accompanying claims).

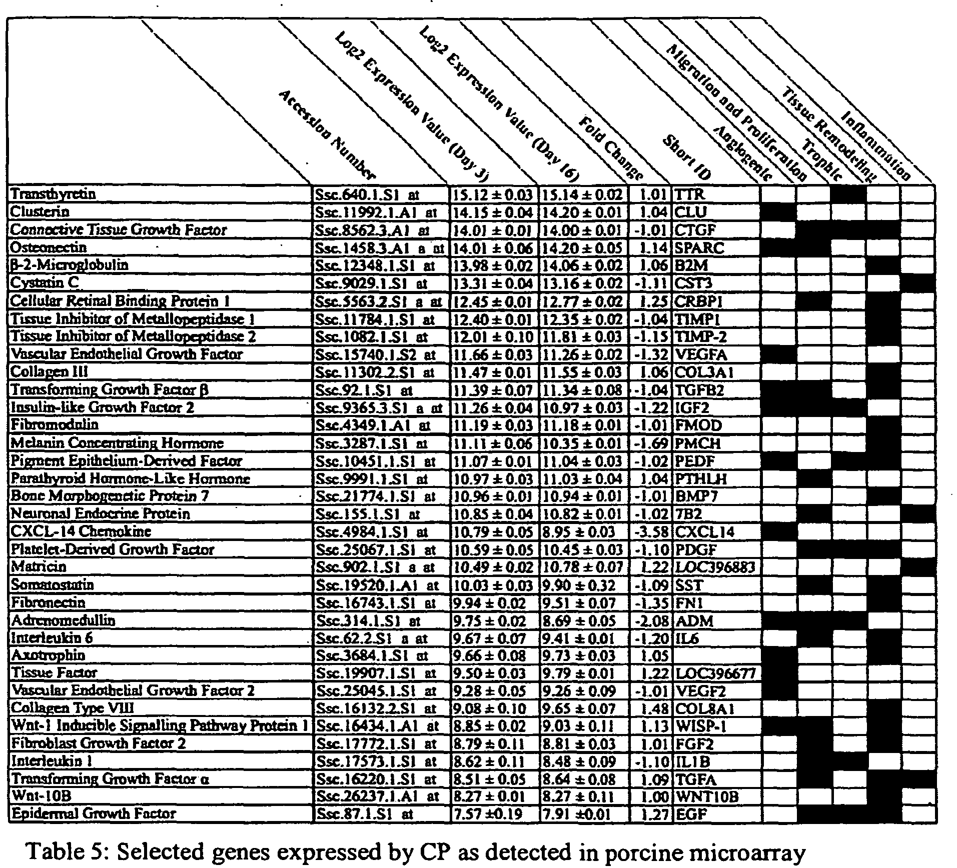

**Table 6: . Selected CP genes that change between day 3 and 16 in ENDO-SFM**

| **Day 3** | **Day 16** | **Fold Change** | **p-value** | **Gene ID** | **Description** |
|---|---|---|---|---|---|
| 10.70 | 7.17 | -11.56 | 0.015 | FABP4 | fatty acid binding protein 4, adipocyte |
| 10.10 | 6.64 | -10.98 | 0.011 | LOC396873 | Haptocorrin |
| 8.83 | 5.69 | -8.76 | 0.043 | TMOD3 | Tropomodulin 3 (ubiquitous) |
| 10.85 | 7.88 | -7.86 | 0.001 | TMOD3 | Tropomodulin 3 (ubiquitous) |
| 9.75 | 7.05 | -6.49 | 0.005 | TMOD3 | Tropomodulin 3 (ubiquitous) |
| 8.46 | 5.93 | -5.81 | 0.027 | C10G | Complement C1qC |
| 8.14 | 5.89 | -4.76 | 0.037 | LOC396873 | haptocorrin |
| 6.80 | 4.78 | -4.08 | 0.039 | CCR1 | chemokine (C-C motif) receptor 1 |
| 8.43 | 6.53 | -3.71 | 0.004 | C1QB | complement component 1, q subcomponent, B chain |
| 7.95 | 6.18 | -3.43 | 0.017 | GP91-PHOX | NADPH oxidase heavy chain subunit |
| 8.86 | 7.33 | -2.88 | 0.040 | PLUNC | Patate, lung and nasal epithelium carcinoma associated |
| 6.83 | 5.39 | -2.71 | 0.046 | LPL | Lipoprotein lipase |
| 9.77 | 8.40 | -2.59 | 0.020 | LPL | Lipoprotein lipase |
| 7.27 | 5.92 | -2.56 | 0.017 | TYROBP | TYRO protein tyrosine kinase binding protein |
| 8.70 | 7.35 | -2.54 | 0.019 | LPL | lipoprotein lipase |
| 7.91 | 6.60 | -2.47 | 0.024 | C1QA | complement component 1, q subcomponent, A chain |
| 11.93 | 10.64 | -2.45 | 0.011 | TXNIP | Thioredoxin interacting protein |
| 7.46 | 6.32 | -2.21 | 0.013 | PU.1 | transcription factor PU.1 |
| 12.40 | 11.33 | -2.11 | 0.006 | C-JUN | C-JUN protein |
| 9.75 | 8.70 | -2.08 | 0.019 | ADM | edrenomedullin |
| 8.21 | 7.23 | -1.97 | 0.009 | FCER1G | Fc fragment of IgE, high affinity I. receptor for: gamma polypeptide |
| 6.26 | 5.34 | -1.88 | 0.022 | CCNB1 | Cydin B |
| 7.60 | 7.60 | 1.91 | 0.038 | PLANH1 | plasminogen activator Inhibitor I |
| 6.67 | 7.61 | 1.91 | 0.009 | PLAU | plasminogen activator |
| 8.70 | 9.67 | 1.96 | 0.016 | FABP3 | fatty acid binding protein 3. muscle and heart (mammary-derived growth inhibitor |
| 8.24 | 9.23 | 1.98 | 0.013 | CD74 | CD74 antigen |
| 8.33 | 9.34 | 2.01 | 0.041 | RAN | RAN. member RAS oncogene family |
| 7.23 | 8.27 | 2.05 | 0.005 | - | EFNA1 |
| 10.59 | 11.71 | 2.18 | 0.018 | CAPNS1 | Calpain, small subunit 1 |
| 11.78 | 12.91 | 2.19 | 0.003 | INSIG1 | Insulin induced gene 1 |
| 9.88 | 11.00 | 2.19 | 0.022 | IRF1 | Interferon regulatory factor 1 |
| 10.10 | 11.34 | 2.36 | 0.014 | LDLR | Low density lipoprotein receptor |
| 8.22 | 9.46 | 2.36 | 0.012 | ALP | antileukoproteinase |
| 9.36 | 10.68 | 2.50 | 0.048 | RPS17 | Ribosomal protein S17 |
| 7.17 | 8.65 | 2.79 | 0.035 | TYRP1 | Tyrosinase-related protein 1 |
| 8.09 | 9.58 | 2.80 | 0.018 | INSIG1 | Insulin Induced gene 1 |
| 8.28 | 9.80 | 2.88 | 0.017 | GEM | GTP binding protein overexpressed in skeletal muscle |
| 8.86 | 9.42 | 5.91 | 0.003 | PMAIP1 | phorbol-12-myristate-13-acetate-induced protein 1 |

### REFERENCES

1. Sedlarik K.M; The Process of Wound Healing. Wung Forum. Published on-line: hartmann_online, 1994
2. Laurence Rosenberg; Wound Healing, Growth Factors. Published on-line: eMedicine. 15 May 2003
3. Michael Mercandetti; Wound Healing, Healing and Repair. Published on-line: eMedicine. 1 August 2005
4. Anderson, J. E. (1998). Murray L. Barr Award Lecture. Studies of the dynamics of skeletal muscle regeneration: the mouse came back! Biochem Cel Biol 76, 13-26.
5. Poor/Slow Wound Healing. On-line publication: Diagnose-me.com. 22 November 2005.
6. Aleshire SL et al., "Choroid plexus as a barrier to immunoglobulin delivery into cerebrospinal fluid." J Neurosurg. 63:593-7, 1985)
7. Johanson CE et al. (2000), "Choroid plexus recovery after transient forebrain ischemia: role of growth factors and other repair mechanisms." Cell Mol Neurobiol. 20:197-216.
8. Thanos, CG et al., (2007). The in vitro expression and secretion of VEGF from free and alginate-poly-ornithine encapsulated choroid plexus epithelium. Tissue Engineering. 13(4) 747-756.
9. Gillitzer R. and Goebeler M. (2001). Chemokines in cutaneous wound healing. Journal of Leukocyte Biology 69(4): 513-21.
10. Yang L., Scott P. G., Dodd C., Medina A., Jiao H., Shankowsky H. A., Ghahary A. and Tredget E. E. (2005). Identification of fibrocytes in postburn hypertrophic scar. Wound Repair and Regeneration 13(4): 398-404.
11. Gillitzer R and Goebeler M, (2001). Chemokines in cutaneous wound healing. Journal of Leukocyte Biology. 69,513-21
12. Bhogal RK, Stoica CM, McGaha TL and Bona CA. Molecular aspects of regulation of collagen gene expression in fibrosis. (2005) Journal of Clinical Immunology 25(6) 592-603.

### Cited Patent Documents

WO 00/66188, WO 06/132548, US 5973007, US 6322804.

### INDUSTRIAL APPLICATION

The present invention provides compounds according to the claims for use in improving wound healing by administering thereof either systemically or locally wherein the compound comprises CP secreted proteins.

## Claims

1. A method of manipulating the profile of choroid plexus (CP) secreted proteins from CP cells in vitro comprising the steps:
a) culturing CP cells in a basal growth medium in vitro;
b) adding cellular matrix proteins to the culture medium to initiate the formation of CP capsuloids;
c) culturing the capsuloids for a time sufficient to increase the number of secretory vesicles in the capsuloids; and
d) producing CP conditioned media containing an altered CP secreted protein profile compared to CP cells cultured in basal growth media alone.

2. CP conditioned media produced by the method of claim 1.

3. A therapeutic composition comprising CP conditioned media of claim 2 for use in improving wound healing by decreasing accumulation of collagen at a wound site in a human or non-human patient or by decreasing accumulation of fibroblasts at a wound site in a human or non-human patient.

4. CP conditioned media as claimed in claim 2 for use in improving wound healing in a human or non-human patient by decreasing accumulation of collagen at a wound site in a human or non-human pantient by decreasing accumulation of fibroblasts at a wound site in a human or non-human patient.

## Patentansprüche

1. Ein Verfahren zur Manipulation des in vitro Profils von vom Coroid Plexus (CP) abgesonderter Proteine aus CP-Zellen, das folgende Schritte umfasst:
a. Kultivieren von CP-Zellen in einem basalen Wachstumsmedium in vitro,
b. Anreichern des Nährmediums mit Zellmatrix-Proteinen zur Anregung der Bildung von CP-Kapseln,
c. Kultivieren der Kapseln für eine zur Vermehrung sekretorischer Vesikel in den Kapseln ausreichende Dauer,
d. Herstellen von CP-konditioniertem Medium mit einem veränderten vom CP abgesonderten Protein Profil gegenüber alleine im basalen Wachstumsmedium kultivierten CP-Zellen.

2. CP-konditioniertes Medium hergestellt nach einem Verfahren nach Anspruch 1.

3. Therapeutische Zusammensetzung umfassend CP-konditioniertes Medium nach Anspruch 2 für die Verwendung zur Verbesserung der Wundheilung durch Verringern der Ansammlung von Kollagen an einer Wundstelle bei einem menschlichen oder nichtmenschlichen Patienten oder durch Verringern der Ansammlung von Fibroblasten an einer Wundstelle bei einem menschlichen oder nichtmenschlichen Patienten.

4. CP-konditioniertes Medium nach Anspruch 2 für die Verwendung der Verbesserung der Wundheilung bei einem menschlichen oder nichtmenschlichen Patienten durch Verringern der Ansammlung von Kollagen an einer Wundstelle oder durch Verringern der Ansammlung von Fibroblasten an einer Wundstelle.

## Revendications

1. Méthode de manipulation du profil des protéines secrétées par les plexus choroïdes (PC) à partir de cellules de PC *in vitro* selon les étapes suivantes :
a. culture de cellules de PC dans un milieu de culture basal *in vitro* ;
b. ajout de protéines matricielles cellulaires au milieu de culture pour initier la formation de capsuloïdes de PC ;
c. culture des capsuloïdes pendant le temps nécessaire à l'augmentation du nombre des vésicules sécrétoires dans les capsuloïdes ; et
d. production de milieux conditionnés par les PC qui contiennent un profil altéré de protéines secrétées par les PC par rapport aux cellules PC cultivées uniquement dans un milieu de culture basal.

2. Milieux conditionnés par les PC produits selon la méthode décrite dans la revendication 1.

3. Composition thérapeutique comprenant les milieux conditionnés par les PC décrits dans la revendication 2 à utiliser pour améliorer la cicatrisation par une réduction de l'accumulation soit du collagène soit des fibroblastes dans la plaie chez un patient humain ou non-humain.

4. Composition thérapeutique comprenant ses milieux conditionnés par des PC tels que décrits dans la revendication 2 à utiliser pour améliorer la cicatrisation chez un patient humain ou non-humain en réduisant l'accumulation soit du collagène soit des fibroblastes dans la plaie chez un patient humain ou non-humain.
